# EUROPEAN PATENT APPLICATION

(11) **EP 1 564 292 A1**
(43) Date of publication of application: **17.08.2005**
(21) Application number: 04105588.0
(22) Date of filing: 19.01.2001
(51) Int. Cl.: C12N 15/11, A61K 48/00, G01N 33/50

(54) **Pharmaceutical compositions and methods of treatment based on VEGF antisense oligonucleotides**

(30) Priority: 19.01.2000 US 487023
(62) Divisional of application: 01942565.1
(71) Applicant: Gill, Parkash, S., Agoura, CA 91301 (US)
(72) Inventor: Gill, Parkash, S., Agoura, CA 91301 (US); Masood, Rizwan, San Gabriel, CA 91775 (US)
(74) Representative: Bergstrand, Mikael Gudmundsson

(57) **Abstract**

This invention relates to compositions and methods for inhibition of abnormal proliferation of cells or angiogenesis. More particularly this invention provides VEGF antisense oligonucleotides capable of inhibiting proliferation of cancer cells or angiogenesis or combinations thereof.

## Description

### 1. FIELD OF INVENTION

This invention relates to the inhibition of angiogenesis and growth of neoplastic cells. More specifically this invention relates to vascular endothelial growth factor (VEGF) antisense oligonucleotides which inhibit the expression of VEGF and to methods for inhibiting growth of cancer cells or angiogenesis which employ these antisense oligonucleotides.

### 2. BACKGROUND OF INVENTION

VEGF was first discovered as a molecule that is a secreted protein that was capable of modulating a nimber of biological processes. For example, VEGF *in vitro* induces the growth of endothelial cells and induces migration of endothelial cells; VEGF induces new vessel formation in model systems, such as the chick chorioallantoic membrane and the rat or rabbit cornea avascular zone; and VEGF induces permeability of the existing blood vessels, in model systems, such as the mice of guinea pig skin vessels. It was later shown that a number of tumor cells produce VEGF and the secreted protein induces the regional blood vessels to produce more blood vessel network (i.e., angiogenesis) to support the tumor growth and metastasis. In addition, inhibition of VEGF function was shown to reduce the growth potential of tumor explants in immunodeficient mice

VEGF functions through the cognate tyrosine kinase receptors, Flt-I/VEGFR-1 and Flk-1/KDR/VEGFR-2. Fit-1 is an intermediate affinity receptor and Fik-1/KDR is a low affinity receptor Expression of both receptors results in high affinity binding of the homodimer of VEGF to the target cells Signal transduction for endothelial cell proliferation, however, occurs through Flk-1/KDR only. VEGF binds with high affinity to its cognate receptors flt-1/VEGFR-1, flk-1/KDR/VEGFR-2 and neuropilin-1 (de Vries, C, et al.,(1992) *Science* 255, 989-91; Terman, B. I. et al., (1992) *Biochem Biophys Res Commun* 187, 1579-86; Soker, S. et al., (1998) *Cell* 92, 735-45). VEGFR-2 is responsible for mitogenic signaling (Waltenberger, J. et al, (1994) *J Biol Chem* 269, 26988-95), while VEGFR-1 participates in cell migration (Barleon, B. et al., (1996) *Blood* 87, 3336-43; Clauss, M. et al., (1996) *J Biol Chem* 271, 17629-34; Wang, D. et al., (2000) *J Biol Chem* 275, 15905-15911). Induced expression of VEGFR-2 in cell lines of non-endothelial cell types does not respond to VEGF mediated mitogenic response (Takahashi, T. & Shibuya, M. (1997) *Oncogene* 14, 2079-89) suggesting that only the endothelial cells are configured to carry mitogenic VEGF signal to the nucleus.

VEGF is expressed as four different splice variants. VEGF 165 and VEGF 121 are secreted proteins. Four other members of the VEGF family have been described recently. These include VEGF-B, VEGF-C, VEGF-D, and placental derived growth factor (PIGF). PIGF has 47% homology to VEGF and binds to Flt-1 as a homodimer or a heterodimer with VEGF. VEGF-B is a 167 amino acid secreted protein and has 43% and 30% homology with VEGF and PIGF. VEGF-C also called VEGF related protein (VRP) has 32% and 27% homology to VEGF and PIGF. It binds to Flt-4 as a homodimer and to Flk-1/KDR as a VEGF heterodimer.

VEGF is also regulated by several factors including hypoxia (VEGF expression is increased by hypoxia as noted in the deepest part of the tumor), cytokines such as IL-1 and IL-6, activation of certain oncogenes (Ras, Raf, Src), and loss-of-function mutations of p53 and the Von Hippel Lindau genes (Enholm, B. et al., (1997) *Oncogene* 14, 2475-83; Okajima, E. & Thorgeirsson, U. P. (2000) *Biochem Biophys Res Commun* 270, 108-11; Mukhopadhyay, D. et al.,(1995) *Cancer Res* 55, 6161-5; Mukhopadhyay, D. et al., (1995) *Nature* 375, 577-81; Rak, J. et al., (1995) *Cancer-Res* 55, 4575-80; Siemeister, G. et al (1996) *Cancer Res* 56,2299-301). Elevated tumor or serum VEGF levels are in many cases predictive of poor survival (Moriyama, M. et al., (1997) *Oral Oncol* 33, 369-74; Maeda, K. et al., (1999) *Cancer* 86, 566-71; Maeda, K. et al., (1996) *Cancer* 77,858-63; Linderholm, B, et al., (2000) *Int J Cancer* 89,51-62; Li, X. M. et al., (1999) *JExp Clin Cancer Res* 18, 511-7; Hida, Y. et al., (1999) *Anticancer Res* 19, 2257-60; Fine, B. A. et al., (2000) *Gynecol Oncol* 76, 33-9; Aguayo,A. etal., (1999) *Blood* 94, 3717-21; Crew, J.P. etal., (1997) *Cancer Res* 57, 5281-5; El-Assal, O. N, et al., (1998) *Hepatology* 27, 1554-62, Paradis, V. et al., (2000) *Virchow) Arch* 436, 351-6; Smith, B. D. et al., *J Clin Oncol* 18, 2046-52).

Angiogenesis is the process whereby new blood vessels sprout from existing vessels in response to local stimuli. These primarily consist of the release of angiogenic factors, activation of metaHoproteases to break down extracellular matrix, followed by remodeling. VEGF is pre-eminent in blood vessel formation, for example, loss of only one allele in knockout mice causes embryonic death (Ferrara, N. et al., (1996) *Nature* 380, 439-42; Carmeliet, P, et al., (1996) *Nature* 380, 435-9). Likewise, the VEGF receptors were also demonstrated to be essential for blood vessel formation by gene knockout in mice (Fong, G. H. et al., (1995) *Nature* 376, 66-70; Shalaby, F. etal., (1995) *Nature* 376, 62-6). The switch to the angiogenic phenotype is crucial in both tumor progression and metastasis (Fidler, I, J, & Ellis, L. M, (1994) *Cell* 79, 185-8). VEGF is a key factor in nearly all human tumors (Dvorak, H, F. ,et al., (1995) *Am J Pathol* 146, 1029-39; Senger, D. R., et al., (1993) *Cancer Metastasis Rev*12, 303-24). Heightened expression of VEGF receptors in the endothelial cells of tumor vasculature further attests to the significance of VEGF in tumor angiogenesis (Chan, A. S. et al., (1998) *AmJ Surg Pathol* 22, 816-26; Leung, S. Y. etal., (1997) *Am J Surg Pathol* 21, 941-50), As a result of the role that VEGF plays in angiogenesis and neoplastic proliferation, there is a great need for agents capable of inhibiting VEGF. Agents capable of inhibiting angiogenesis and/or neoplastic proliferation would have tremendous therapeutic utility in cancer or any other disease involving pathological angiogenesis or abnormal cellular proliferation.

### 3. SUMMARY OF THE INVENTION

This invention relates, in general, to compositions and methods for inhibition of cancer cells or angiogenesis or a combination thereof. More particularly this invention is directed to VEGF antisense oligonucleotides and methods of inhibiting proliferation of cancer cells or angiogenesis or combinations thereof using the VEGF antisense oligonucleotides. This invention is further directed to screening and prognostic assays, as well as kits comprising the VEGF antisense oligonucleotides.

It is an object of this invention to provide VEGF antisense oligonucleotides and modified VEGF antisense oligonucleotides which inhibit VEGF expression.

It is another object of this invention to provide VEGF antisense oligonucleotides and modified VEGF antisense oligonucleotides which inhibit proliferation of cancer cells or cancer cell viability and/or angiogenesis.

It is yet another object of this invention to provide methods of using the VEGF antisense oligonucleotides and modified VEGF antisense oligonucleotides to inhibit VEGF expression.

It is another object of this invention to provide a method of using the VEGF antisense oligonucleotides and modified VEGF antisense oligonucleotides to inhibit proliferation of cancer cells or cancer cell viability and/or angiogenesis.

Another object of this invention is to provide a method of inhibiting VEGF expression in a subject by administering the VEGF antisense oligonucleotides or modified VEGF antisense oligonucleotides either alone or in conjunction with one or more other agents.

Yet another object of this invention is to provide a method of inhibiting angiogenesis or cancer cell proliferation in a subject by administering the VEGF antisense oligonucleotides or modified VEGF antisense oligonucleotides either alone or in conjunction with one or more other agents.

It is another object of this invention to provide pharmaceutical compositions for use in the methods described herein.

It is another object of this invention is to provide a method of screening for new inhibitors of VEGF using cells exhibiting autocrine VEGF growth activity (e.g., a cell line that produces produces and uses VEGF for its own growth, such as certain KS cell lines, ovarian cell lines, melanoma, cell lines).

Another object of this invention is to provide a prognostic assay for a subject with a disease exhibiting pathological angiogenesis and/or proliferation of cancer cells by assessing the VEGF receptor status of the tumor in the diseased tissue or by evaluating the ability of the VEGF antisense oligonucleotides and modified VEGF antisense oligonucleotides to inhibit cellular proliferation or viability in the diseased tissue (e.g., primary tumor cell cultures).

It is a further object of this invention to provide a kit or drug delivery system comprising the compositions for use in the methods described herein.

### 4. BRIEF DESCRIPTION OF DRAWINGS

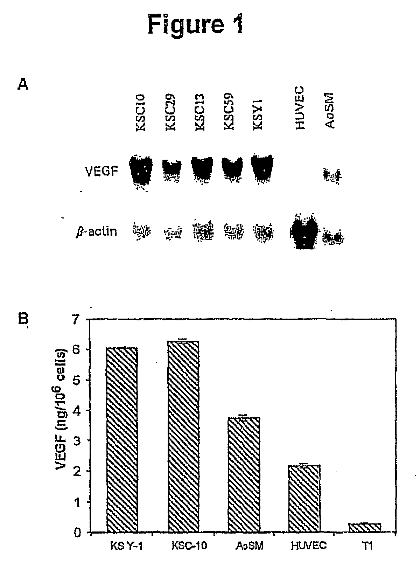

Figure 1 shows that KS cells produce VEGF mRNA and protein at high levels when compared to other cell types such as fibroblasts, endothelial cells, and vascular smooth muscle cells. (A) Equal number of cells were used to extract total RNA, and Northern blot analysis were performed for VEGF. In addition the relative amount of RNA was assessed by probing the membranes for beta-actin, a house keeping gene. (B) Equal number of cells were grown in 25 cm² flasks and the supernatants were collected after 24 hr, and the VEGF levels were measured by ELISA.

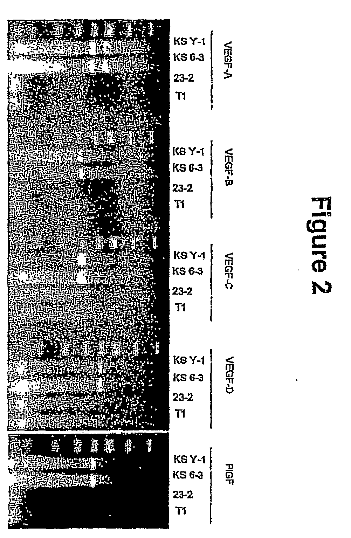

Figure 2 illustrates expression of VEGF family members in KS and other tumor cell lines. VEGF expression is observed in KS cell lines, whereas no expression is observed in a B cell (23-2) and in a fibroblast cell line (T1). Expression. The RT-PCR product of VEGF members are seen on agarose gel. Kaposi's sarcoma cell line KSY-1 and cell line KS 6-3 express VEGF-A, VEGF-B, VEGF- C, VEGF- D, and placental growth factor (PIGF) in contrast to B lymphoma (23-2) and fibroblast (T1) cell lines that do not express these genes.

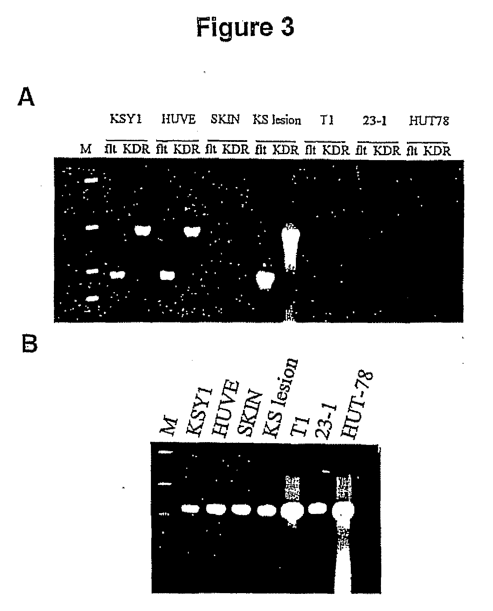

Figure 3 (A) shows that KS cells lines and primary KS tumors express both VEGF receptors (Ftk-1/KDR and Flt -1). Several other cell lines including T-cell lines, B-cell lines and fibroblast cell lines were tested and none of which had any evidence of VEGF receptor expression. Normal human endothelial cells (HUVEC), as expected, served as positive controls. KS cells and control cells were grown in 75 cm² flasks until near confluence. Total cellular RNA was solubilized in guanidinium thiocyanate and cDNA synthesized. Using a specific primer pair for each of the two VEGF receptors, the mRNA transcripts were amplified and the products were resolved on agarose gel. (B) Integrity of the mRNA was confirmed by the demonstration of house keeping gene (-actin) levels in the same cell lines.

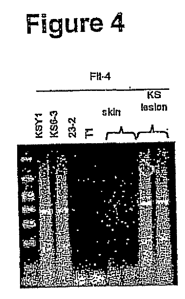

Figure 4 demonstrates the expression of Flt 4 (VEGF-C receptor) in KS, other cell lines, and also the pair of samples of skin and KS lesions from the same patient. The figure shows RT-PCR product on agarose gel. Kaposi 's sarcoma cell lines KSY-1, KS 6-3, express PIGF and Flt-4. In contrast B lymphoma (23-2) and fibroblast (T1) cell lines do not. Similarly, Flt-4 was expressed by the KS tumor lesion and not the skin from the same patient.

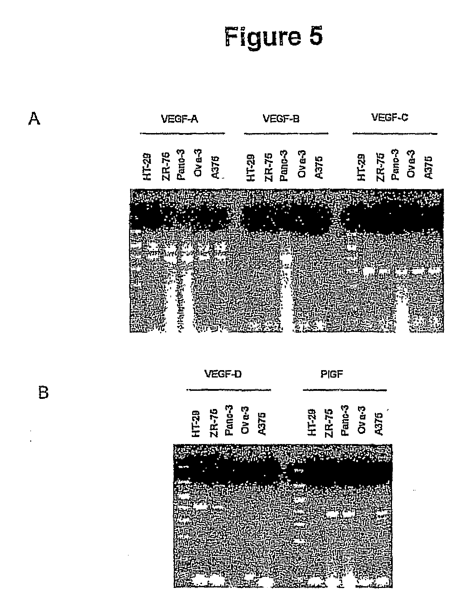

Figure 5 (A) shows that many of the tumor types, including colon (HT-29), breast (ZR-75), pancreas (pane), ovarian (ova-3), and melanoma (A-375), express VEGF-A and VEGF-C (Fig 5A), while expression of the other VEGF family members is heterogeneous (Figs, 5A and 5B).

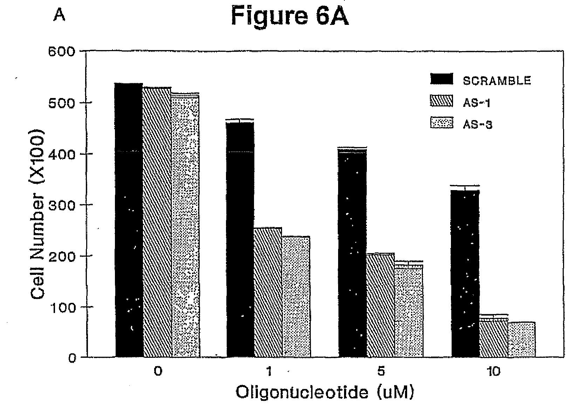

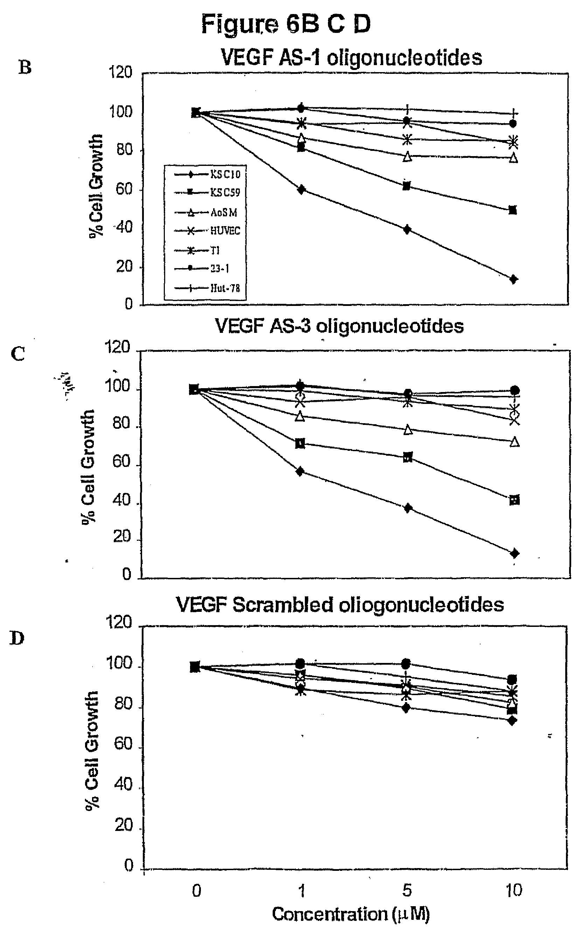

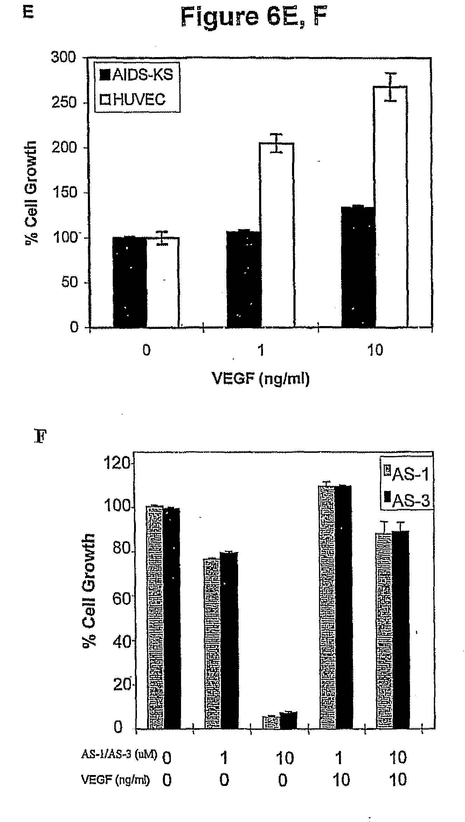

Figure 6 shows that VEGF is an autocrine growth factor for KS tumor cells. Equal number of cells were plated and treated with different concentrations of AS-1/Veglin-1 (SEQ ID NO:1), AS-3/Veglin-3 (SEQ ID NO:2) or scrambled oligonucleotides (SEQ ID NO:30). The cell numbers represent the median of the experiments done in triplicates. (B) shows identical experiments done with several different cell types including KS cells (KSC-10, KS-59), human aortic smooth muscle cells (AoSM), human umbilical vein endothelial cells (HUVEC), fibroblast (T1), B lymphoma cells (23-1), T lymphoma cell line (HUT-78) using AS-1/Veglin-1 (SEQ ID NO:1), AS-3/Veglin-3 (SEQ ID NO:2), and scrambled oligonucleotides (SEQ ID NO:30). Figure 6E shows the effect of exogenous recombinant VEGF on HUVEC or KS cell proliferation., Recombinant VEGF (R&D Systems, Minneapolis, MN) was added to cells on day I and 3, and the cells were counted on day 5. The results represent the median of experiments done in triplicates. HUVEC showed dose dependent increase in cell proliferation while the response of KS cells was markedly blunted, possibly due to the occupancy of VEGF receptors by the endogenously produced ligand. Figure 6F shows the inhibition of endogenous VEGF production in KS cells by AS-1/Veglin-1 (SEQ ID NO:1) or AS-3/Veglin-3 (SEQ ID NO:2) makes cells sensitive to the exogenous VEGF. KS cells were treated with either SEQ ID NO: 1 or 2 alone at various concentrations or with SEQ ID NO: or 2 combined with VEGF The results represent median of the experiments done in triplicates.

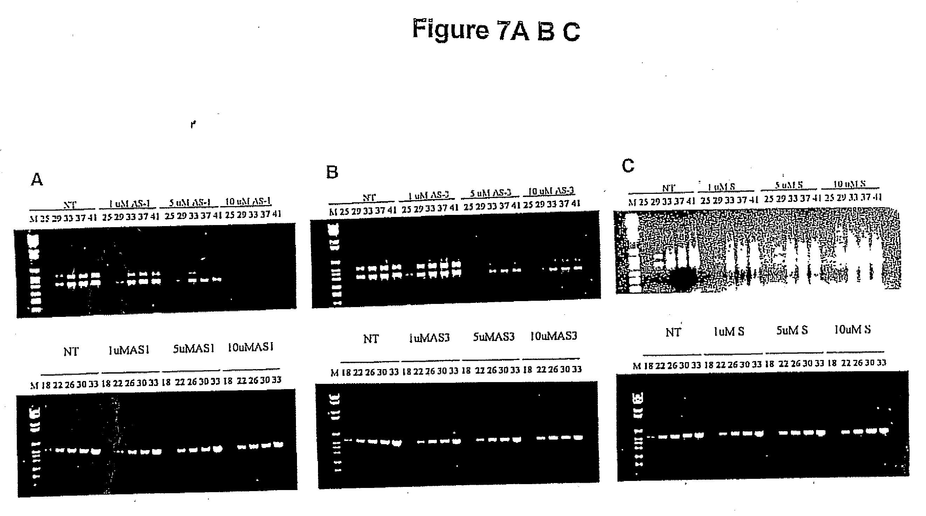

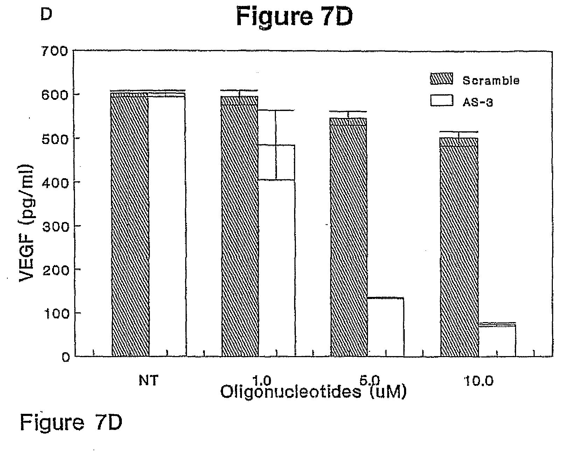

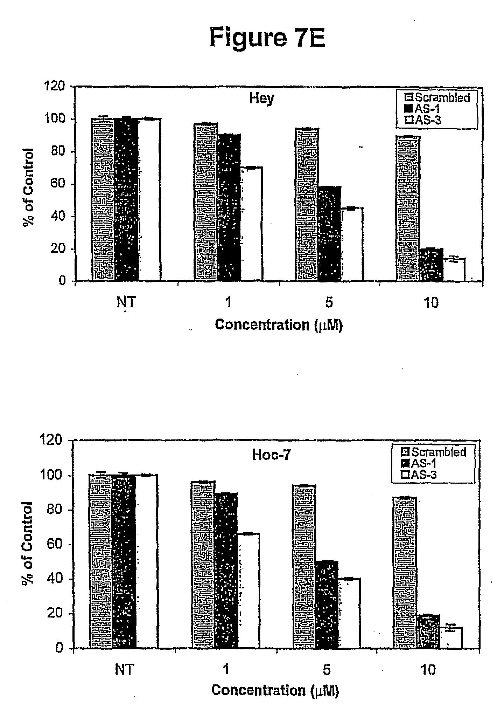

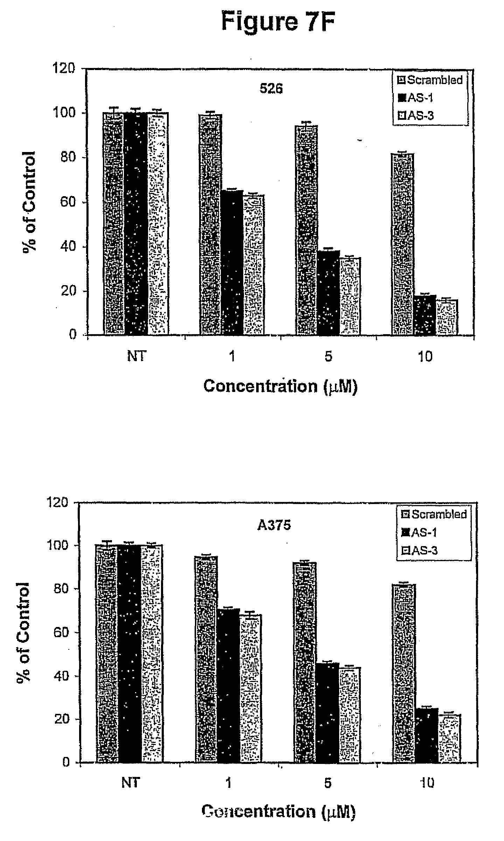

Figure 7 illustrates specificity of VEGF antisense oligonucleotides. KS cells were treated at various concentrations with either AS-1/Veglin-1 (SEQ ID NO:1) (A), AS-3/Veglin-3 (SEQ ID NO:2) (B), or scrambled oligonucleotide (SEQ ID NO: 30) (C). RT-PCR was done for VEGF mRNA (top) or -actin (bottom). PCR products after various cycles of amplification (25-41) were resolved on agarose gel. Figure 7D reveals that AS-3/Veglin-3 (SEQ ID NO:2) but not scrambled oligonucleotides reduced the production of VEGF and the effect was dose dependent. Equal number of KS cells were plated in triplicate wells and treated with oligonucleotides. Supernatants were collected and assayed for VEGF levels by ELISA (R&D Systems, Minneapolis, MN). Figure 7E shows the cell proliferation assay with the oligonucleotides in two different ovarian carcinoma cell lines (both scrambled (SEQ ID NO:30) and antisense oligonucleotides AS-1 (SEQ ID NO:1) and AS-3 (SEQ ID NO:2). Both antisense oligonucleotides inhibited growth of ovarian carcinoma cell lines (Hey top panel, Hoc-7 bottom panel), while scrambled oligonucleotides had no effect. Similar results were seen in Melanoma cell lines (Figure 7 F) 526 in the top panel and A375 in the bottom panel. These cell lines thus express VEGF receptors and use VEGF for autocrine growth activity.

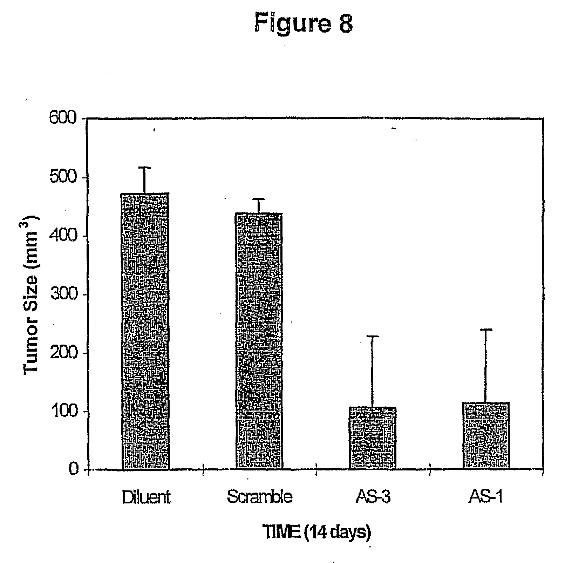

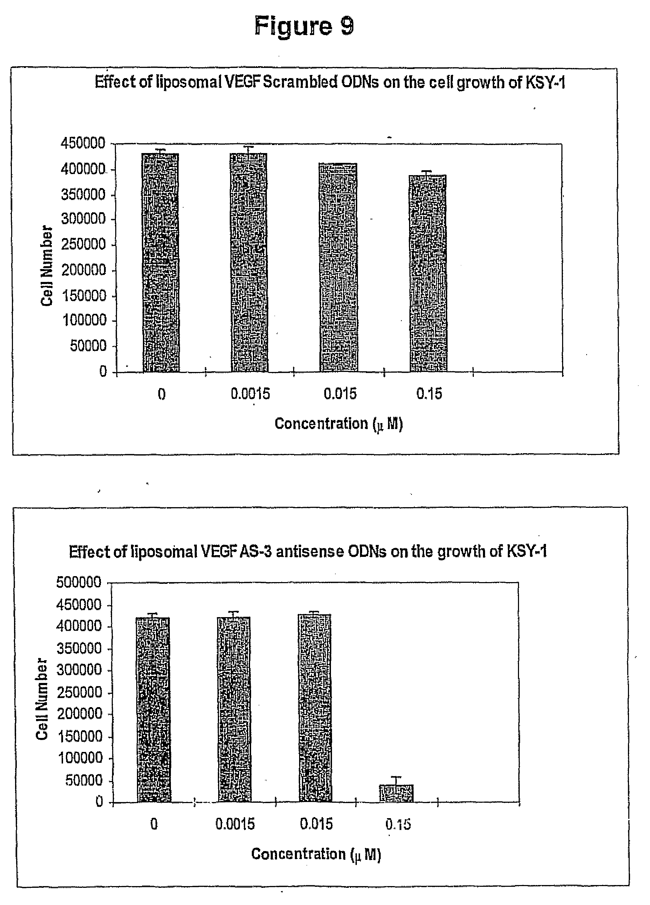

Figure 8 shows that Veglin-1 (SEQ ID NO: 1) and Veglin-3 (SEQ ID NO: 2) are active *in* vivo to inhibit KS tumor growth. Immunodeficient mice bearing KS explants were treated with Veglin-1 (SEQ ID NO: 1) or Veglin-3 (SEQ ID NO: 2) or scrambled oligonucleotides, each given intraperitoneally daily for five days beginning one day after the tumor explants. The tumors were then allowed to grow for a total of 14 days. The tumor sizes were measured. The animals were then sacrificed and the tumors were removed and measured again.

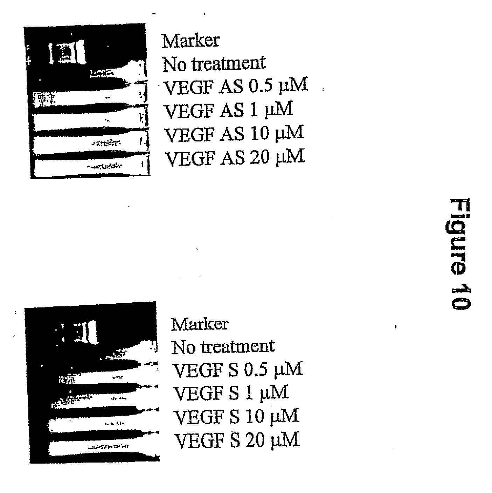

Figure 9 illustrates the effects of liposomal encapsulation of Veglin-1 (SEQ ID NO: 1) and Veglin-3 (SEQ ID NO: 2). We have shown previously that liposomes deliver higher amounts of the drugs into the KS tumor cells than do free drugs. We thus encapsulated scrambled oligonucleotides and Veglin-3 (SEQ ID NO: 2) in the liposomes and treated the KS cells seeded at equal density in 24 well plates. The cell counts were performed on day 5 and the results are presented as the mean and SE of assays performed in triplicate. Liposomally encapsulated Veglin-3 (SEQ ID NO: 2) induced 50% inhibition of KS cell growth (IC₅₀) at doses 50 fold lower than required for free Veglin-3 (SEQ ID NO: 2).

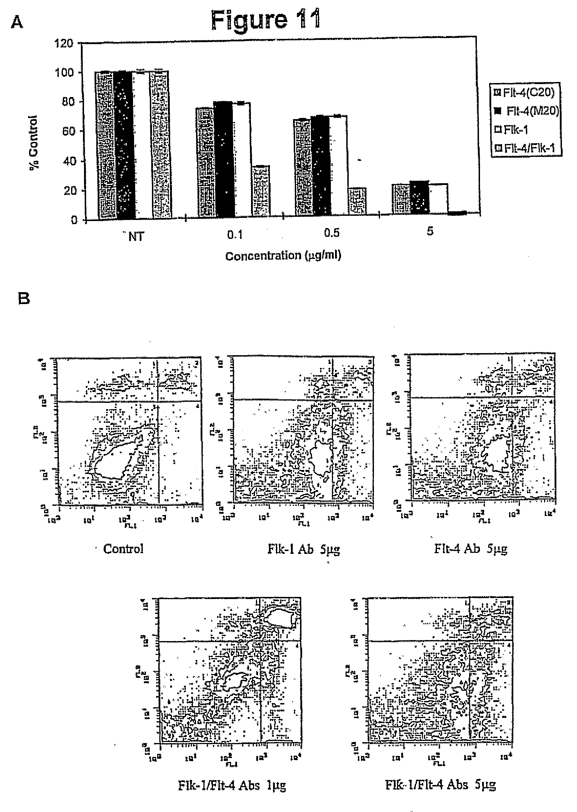

Figure 10 shows that VEGF is a factor necessary for the survival of KS cells, Blocking VEGF production with Veglin-1 (SEQ ID NO: 1) or Veglin-3 (SEQ ID NO: 2) causes cell death in KS cell. KS cells were seeded at equal density in 75 cm² flasks, serum starved for 24 hr and treated with either Veglin-1 (SEQ ID NO: 1) or Veglin-3 (SEQ ID NO: 2) or scrambled oligonucleotide (SEQ ID NO:30), and the cell death was measured by examining the liberation of small DNA fragments (indicative of a specific method of cell death called programmed cell death or apoptosis). The DNA was extracted and size fractionated on the agarose gel.
Figure 11 (A) illustrates the effect of Flk-1 and Flt-4 antibodies (separate and in combination) on KS Y1 cell proliferation. Flk-1 and Flt-4 antibodies were purchased from Santa Cruz Biotechnology, Santa Cruz, CA. KS cells were plated at equal density and treated on day 1 and day 3 with various concentrations of the antibodies. Cell count was performed on day 5. The results represent median of experiments done in triplicates. Figure 11 (B) demonstrates that VEGF receptor antibodies (disruption of VEGF autocrine pathway) induce apoptosis of KS cells. KS cells were treated with various concentrations ofVEGFR-2 (Flk-1) and VEGFR-3 (Flt-4) antibodies for 48 hours. The treated cells were incubated with fluorescein conjugated annexin V and propidium iodide for 15 minutes at room temperature in the dark and analyzed by flow cytometry. Cells undergoing apoptosis stained only with annexin V FITC reagent. The apoptotic cells show the shift of cell population to the right at X axis as shown above.

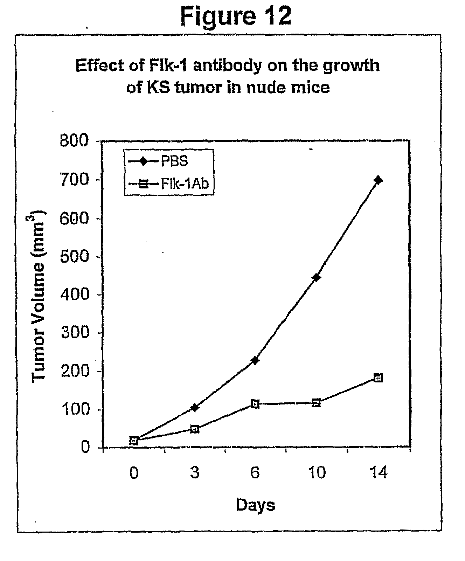

Figure 12 illustrates inhibition of KS tumor growth by anti-VEGFR2 (Flk-1) antibodies. KS Y-1 cells (5x10⁶) cells were inoculated subcutaneously in lower back of Balb/C Nu+/Nu+ athymic mice. After 3 days of tumor growth, 200 ug of Flk-1 antibody was injected intraperitonealy daily for six consecutive days to one group of four mice, and the diluent alone to the control group of four mice. The tumor volume was measured twice a week for two weeks.

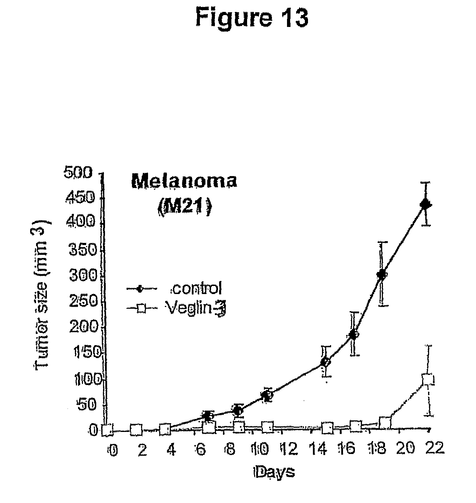

Figure 13 shows the effect of AS-3 (SEQ ID NO: 1) on human melanoma cells *in vivo* Human melanoma cells were inoculated subcutaneously in lower back of Balb/C Nu+/Nu+ athymic mice. Tumor size was measured for control animals receiving a scrambled oligonucleotide (SEQ ID NO: 30) or antisense oligonucleotide (SEQ ID NO: 2).

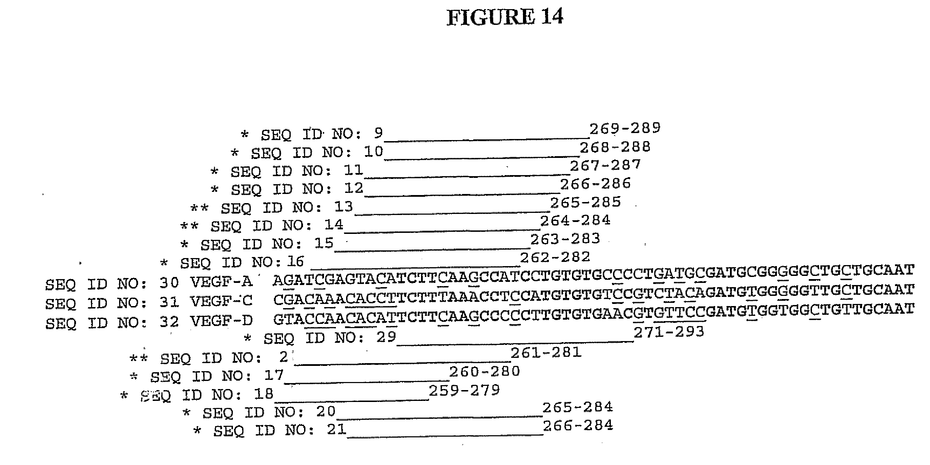

Figure 14 shows the position of selected antisense oligonucleotides denoted by asterisks in Table I relative to the gene sequence for VEGF-A. Asterisks correspond to those listed in Table 1. Individual SEQ ID NOS are to the left of the brackets. Numbers to the right of the brackets represent the VEGF- 165 isoform sequences that the antisense molecules are complementary to. Gene sequence numbers are according to Leung et al., (1989) where numbering started at the translation start site. The sequences of VEGF-A, -C, and -D are aligned, with 3/3 matches indicated by bold faced type, and 2/3 matches by underlining.

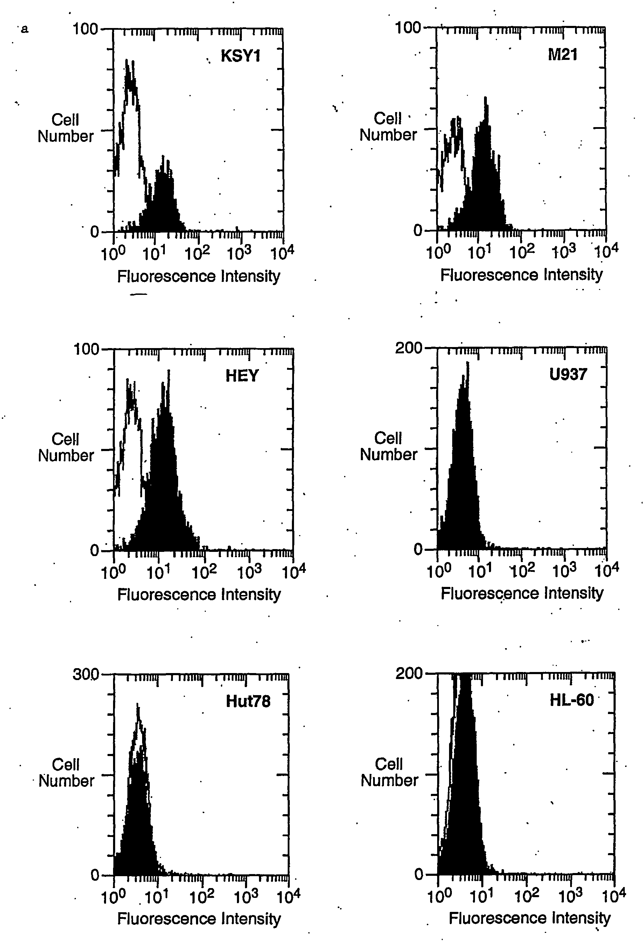

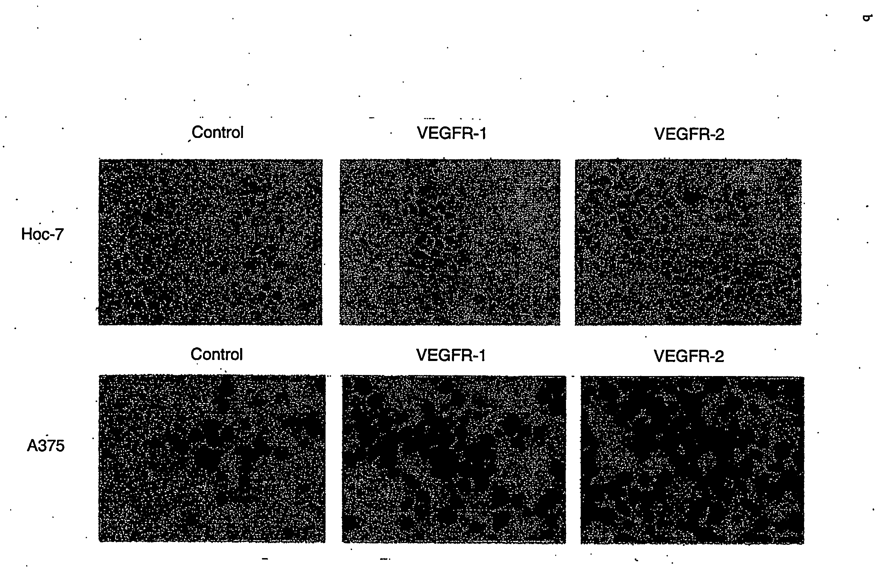

Figure 15 shows expression of VEGFR-2/KDR/flk-1 and VEGFR-1/flt-1 in various tumor cell lines Figure 15 (A). KS Y-1, M21, Hey, U937, HL-60 and HuT 78 cells were incubated with FITC labeled VEGFR-2 antibody as described in the methods and analyzed by flow cytometry. Figure 15 (B). Immunocytochemical staining of Hoc-7 ovarian carcinoma cells and A375 melanoma cells for VEGFR-1 and VEGFR-2. For Hoc-7 brown color is signal and for A375 crimson color is signal. Specificity of immunostaining was demonstrated in both cases by lack of signal with isotype specific controls,

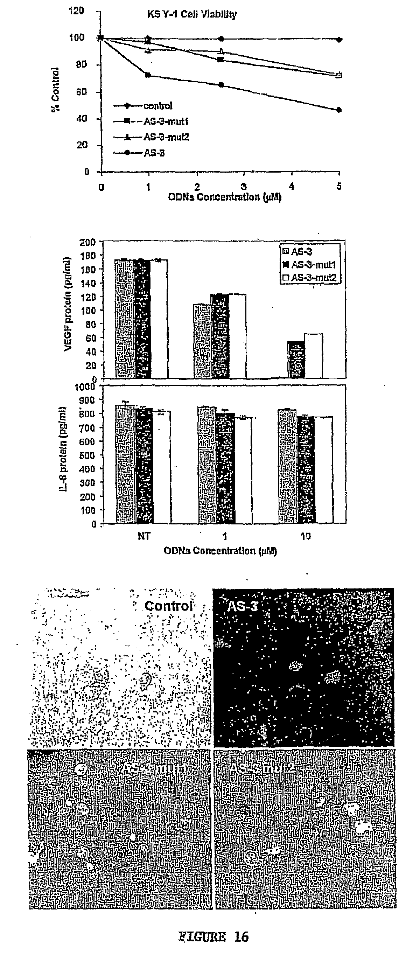

Figure 16 shows VEGF antisense specifically inhibits VEGF Figure 16 (A) Effect of AS-3 and mutant AS-ODNs on the viability of KS Y-1 cells in vitro, Cells were seeded at 1 × 10⁴ cells/well in 24-well plates and treated with the ODNs as indicated on days 1 and 3. Cell viability was performed on day 5 by MTT assay. Results represent the means of quadruplicate samples Figure 16 (B). Effect of AS-3 and mutant AS-ODNs on the production of VEGF and IL-8. Cells were cultured in 2% FCS for these experiments. Cells were treated with various concentrations of the oligonucleotides at hr 0 and 16. The supernatants were collected at hr 24and assayed for VEGF and IL-8 using ELISA kits (R&D Systems, Minneapolis, MN). Results are presented as median of replicate experiments ± SE. C) Fluorescein-tagged ODNs are taken up by KS Y-1 cells in vitro. Overlay images of phase contrast and fluorescein signal of KS Y-1 cells exposed to AS-3m, AS-3m mut1 and AS-3m mut2 (1uM) without cationic lipid or other permeabilizing agent. Control was no treatment (no fluorescent AS-ODN). In each sample there are cells that have taken up AS-ODN (green color) and cells which have no uptake. The number of cells showing fluorescent signal appears similar in each sample. Identical results were seen when the experiments were repeated using melanoma cell line (M21) and ovarian cell line (Hey). The results thus are not limited to one cell line.

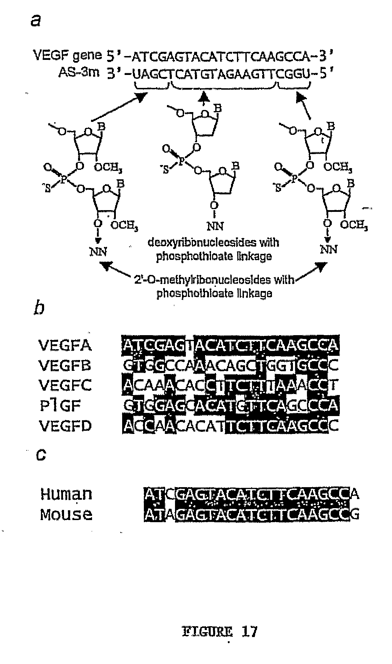

Figure 17 shows VEGF antisense mixed backbone oligonucleotides. Figure 17 (A) Schematic representation of the mixed backbone formulation oligonucleotides, Shown are the human VEGF gene sequence and complementary AS-3m sequences. The chemical structures of the modified bases are shown below. Figure 17 (B) Comparison of the corresponding areas of the VEGF family members. The highlighted bases indicate identity between either VEGF-B, -C, -D or PIGF and VEGF. Homology between the genes is not high in this region Figure 17 (C) Comparison of the sequences in the human and mouse VEGF genes that are complementary to AS-3m. Mouse sequence shown here is nucleotides 288-308 of the sequence reported by Claffey and coworkers (Claffey, K.P. et al (1992) J. Biol. Chem. 267, 16317-2257). Identity is indicated by highlighted blocks.

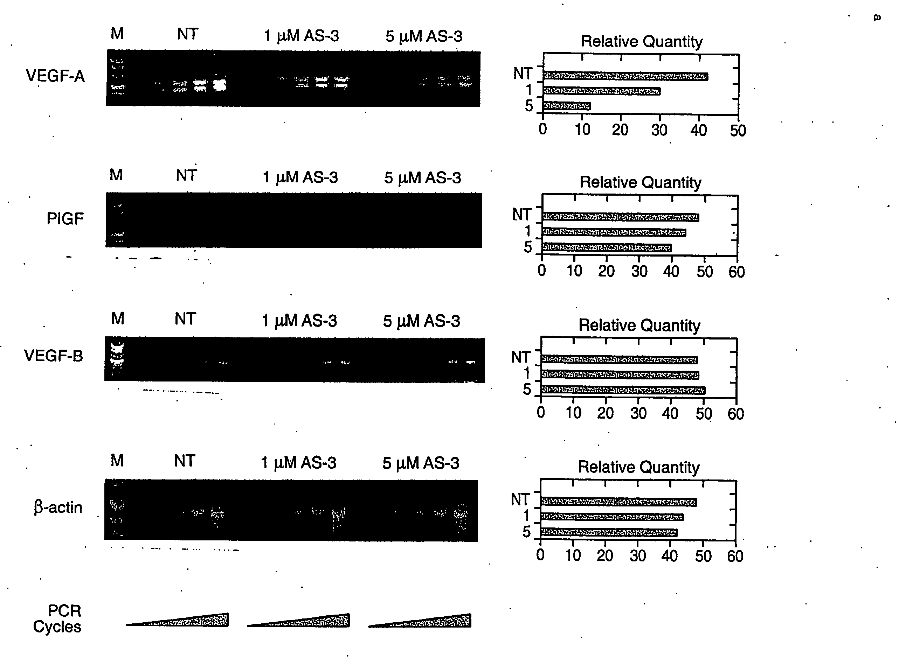

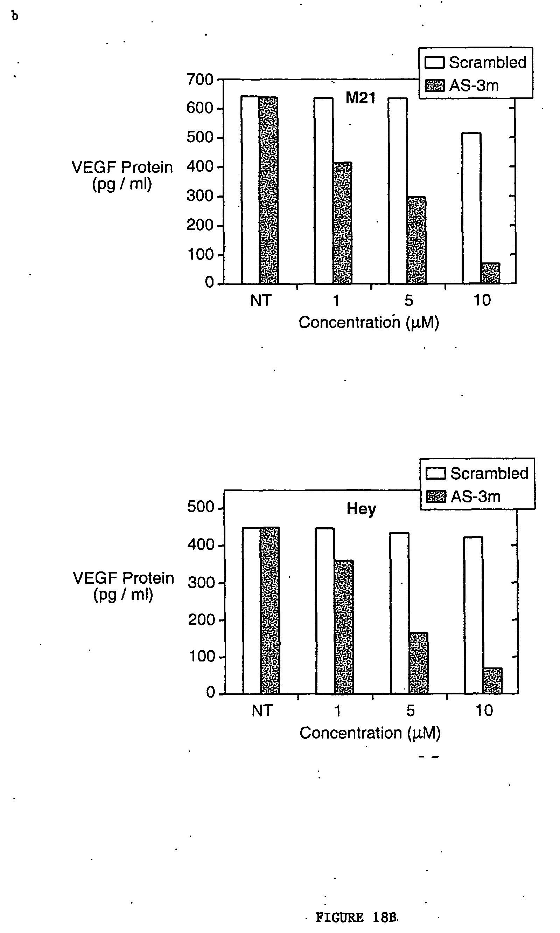

Figure 18 shows mixed backbone antisense AS-3m inhibits VEGF mRNA and protein production. Figure 18 (A) Total RNA was isolated from KS Y-1 cells treated with various concentrations of AS-3m as indicated (NT = not treated). Total RNA was reverse-transcribed to generate cDNA. Aliquots of the reaction mixture were removed at 5-cycle intervals to provide semi-quantitative analysis as described in the methods. Gene specific primers were for VEGF, VEGF-B and PIGF. Intensity of the bands was quantitated and is shown in the graphs on the right. Integrity of RNA in the samples was verified by -actin amplification. Figure 18 (B) Effect of AS-3m on VEGF protein production in two tumorigenic cell lines: human melanoma cell line M21 (left panel) and human ovarian carcinoma cell line Hey (right panel) were treated with VEGF antisense AS-3m and the scrambled MBO at concentrations ranging from 1 to 10 M. Supernatants were collected at 48 h, and VEGF protein was quantitated by ELISA. The results represent the mean ± standard deviation of two separate experiments done in duplicate.

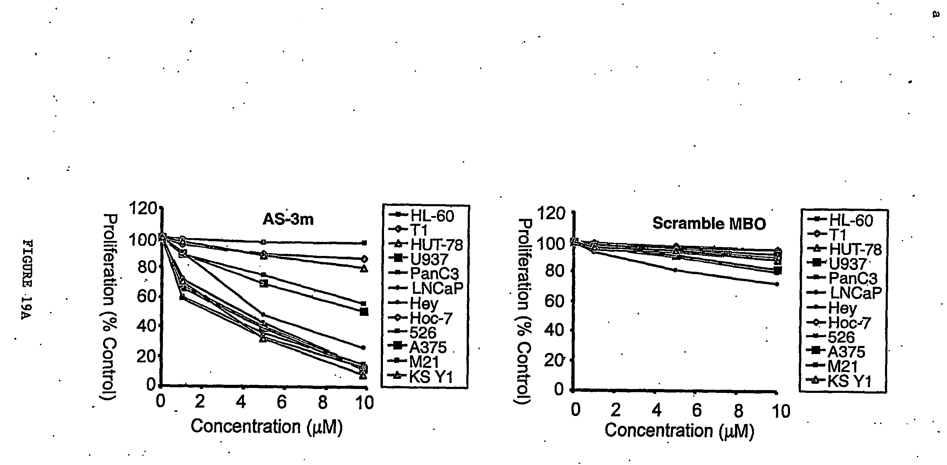

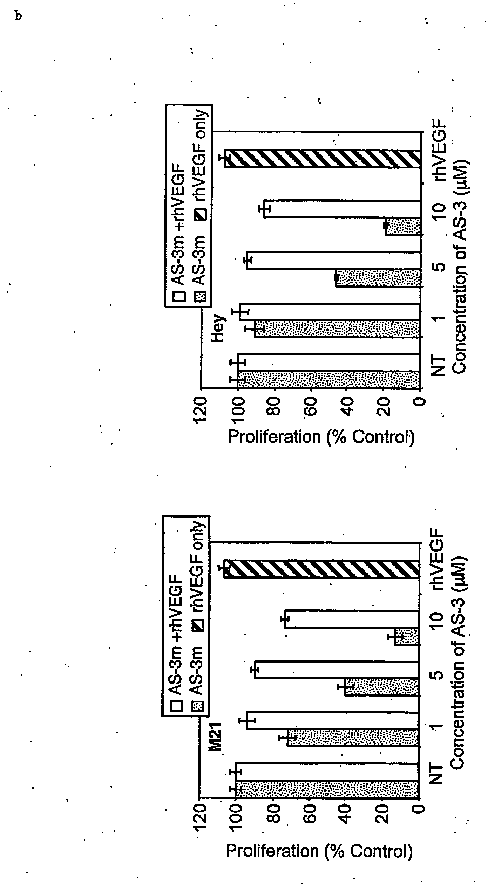

Figure 19 shows mixed backbone antisense AS-3m inhibits cell proliferation in vitro. Cells were seeded at 1 X 10⁴ cells per well in 24 plates and treated with AS-3m (1, 5, 10 M) on days 1 and 3 Figure 19 ( A). Cell viability was performed on day 5 by MTT assay. Results represent the mean ± SD of quadruplicate samples, Specificity of the AS-3 ODN is shown by the lack of significant cytotoxicity in any cell line of the scrambled ODN (right panel). Figure 19 (B) rhVEGF abrogates the effect of VEGF antisense. Cell lines M21 and Hey were seeded as above and were treated with 1, 5 and 10 M of AS-3 alone or with rhVEGF (10 ng/ml) on day 1 and day 2. Cell viability was measured after 72 hours. AS-3m inhibition of cell proliferation in both cell lines (black columns) could be reversed by the presence of VEGF (white columns), which did not have any appreciable effect on the growth of cells (hatched columns). The data represent the mean ± standard deviation of two experiments performed in quadruplicate.

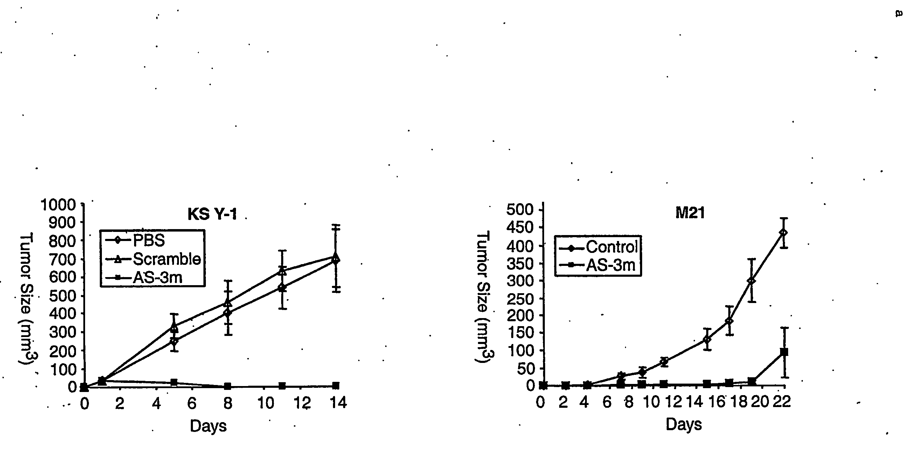

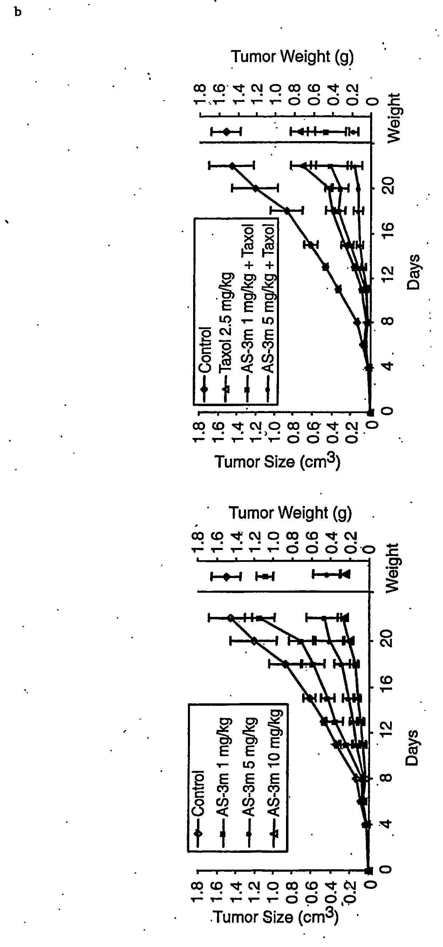

Figure 20 shows the Effect on tumor growth of mixed backbone VEGF antisense oligonucleotides in vivo. Tumor xenografts were initiated by subcutaneous inoculation of cell lines in the lower back of Balb/C/Nu⁺/NU⁺ athymic mice as described in the Methods. Figure 20 (A). Oral administration of AS-3m, Scrambled (S) VEGF oligonucleotides, and diluent (PBS) from the day following KS Y-1 (left panel) and M21 (right panel) xenograft implantation. Dosage was 10 mg/kg daily for 14 days. Figure 19 (B) Effect of combined treatment with AS-3m and chemotherapy (Taxol) on 5-day established M21 tumor xenografts, AS-3m or PBS was injected intraperitoneally daily beginning day 5. Taxol was given i.p. on days 5 and 12 at 2.5 mg/kg. Left hand panel shows dose response to AS-3m alone. Right hand panel shows results of combined treatments. Tumor volumes were measured three times a week. Final tumor weights are shown to the right of the growth curves in each graph. Mice were sacrificed at the completion of the experiment. Data represent the mean ± standard deviation of 6 mice in each group. Experiments were also conducted using human ovarian carcinoma cell line (Hey) implanted in athymic mice. The tumors were allowed to establish for five days before initiation of the treatment with AS-3m. the treatment was given daily i.p. at a dose of 10 mg./kg. The tumor volumes of the treated mice (6 mice) were reduced by more than 805 compared to the controls 96 mice).

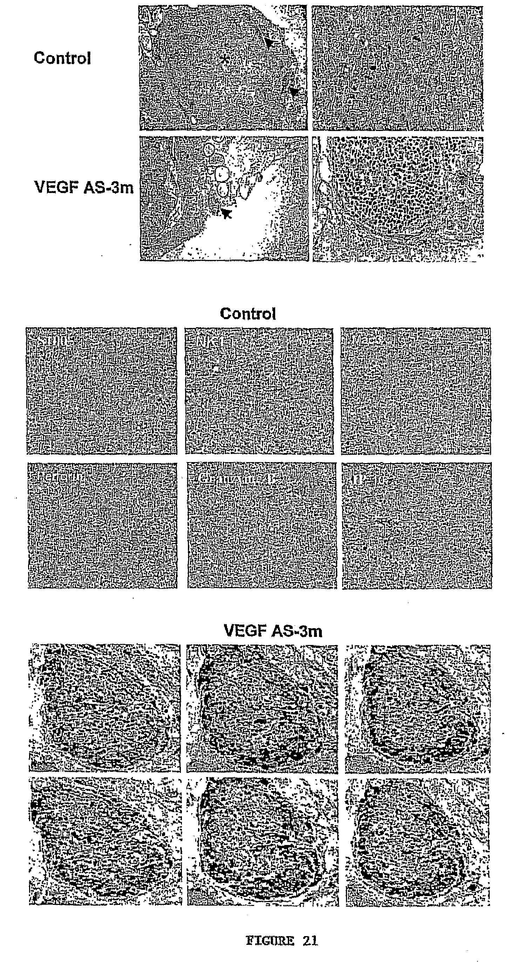

Figure 21. Histology and immunocytochemistry on the orthotopic prostate tumors treated with VEGF-AS.3m. Photomicrographs of H&E stained sections ofPC-3 orthotopic tumors. Figure 21 (A) Top panel reveals prostate gland and the growthof PC-3 human prostate tumor cells within the gland, Control mice treated with the diluent alone (PBS) reveal large tumor (*)ncircled by immune cells (arrow) noted by dense nuclear stain (at lower power) and high mitotic rate in the tumor at higher power. VEGF-AS3 treated mice reveal small tumor nodule within the prostate gland (arrow), showing infiltration with immune cells at higher power. Lower pane reveals Immunostaining with S100 for dendritic cells, NKl.1 for NK cells, Mac3 for activated macrophages, perforin, granzyme B and IP-10. Tumor tissue from VEGF-AS3m treated mouse reveals infiltration with dendritic, NK and macrophage. Expression of perforin, granzyme B, and IP-10 is seen most strongly in regions of immune cell infiltrate while only IP-10 is notable in the control group.

### 5. DETAILED DESCRIPTION OF THE INVENTION

### DEFINITIONS

The term "response" means a halt in the progression and/or a decrease in tumor size. For example, a halt in the progression of KS lesions,

The term "partial response" means a about a 50% reduction in tumor size or load. By way of example in a cancer such as KS a partial response may be a complete flattening of more than about 50% of the raised lesions lasting for four weeks or more in KS

The term "therapeutically effective amount" of a VEGF antagonist, such as a VEGF antisense oligonucleotide, means an amount calculated to achieve and maintain a therapeutically effective level in the tumor, if applied to the tumor, or in the plasma, if administered systematically, so as to inhibit the proliferation of cancer cells and or angiogenesis By way of example, the therapeutic amount be sufficient to inhibit proliferation of more than about 50 percent of cancer cells, such as KS cells, *in vitro.* Of course, the therapeutic dose will vary with the potency of each VEGF antagonist in inhibiting cancer cell growth *in vitro,* and the rate of elimination or metabolism of the VEGF antagonist by the body in the tumor tissue and /or in the plasma.

The term "IC₅₀" means the concentration of substance that is sufficient to inhibit a test parameter (such as, e.g., cell growth, tumor volume, VEGF protein expression, cell viability etc.) by about 50 percent.

The term "antagonist" means a compound that prevents the synthesis of the target molecule or binds to the cellular receptor of the target molecules or an agent that blocks the function of the target molecule.

The term "antisense oligonucleotide" refers to poly nucleotide sequences, which modulate the expression of a gene. Generally, nucleic acid sequences complementary to the products of gene transcription (e.g., mRNA) are designated "antisense", and nucleic acid sequences having the same sequence as the transcript or being produced as the transcript are designated "sense". The antisense compound preferably modulates either gene or protein expression or impairs the function of the protein.

The term "polynucleotide sequence" refers to a stretch of nucleotide residues. The polynucleotide compositions of this invention include RNA, cDNA, genomic DNA, synthetic forms, and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (*e.g,* methyl phosphonates, phosphotriesters, phosphoamidates, carbamates, etc .), charged linkages (e.g., phosphorothioates, phosphorodithioates, etc.), pendent moieties *(e* g, polypeptides), intercalators (e.g, acridine, psoralen, etc.), chelators, alkylators, and modified linkages *(e g.,* alpha anomeric nucleic acids, *etc.)* Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule,

The term "scrambled oligonucleotide" means a sequence of nucleic acid constructed so as to match the nucleic acids content but not the sequence of a specific oligonucleotide.

The term "disease or disorder" refers to a variety of diseases involving abnormal proliferation of cells, such as, for example, vascular endothelial cells. Such diseases include, but are not limited to, proliferative retinopathy (diseases of the eye in which proliferation of the blood vessels cause visual loss), macular degeneration, collagen vascular diseases, skin diseases such as psoriasis and pemphigus, diabetic retinopathy, benign tumors and cancers and precancerous conditions (e.g., premalignant cells).

The term "cancer" includes a myriad of diseases, characterized by inappropriate cellular proliferation of a variety of cell types. Examples include, but are not limited to, ovarian cancer, breast cancer, pancreatic cancer, prostate cancer, melanoma, Kaposi's sarcoma, lung cancer, colon cancer, kidney cancer, prostate cancer, brain cancer, sarcomas, cervical carcinoma, head and neck cancers, brain tumors, such as gliablastoma, and any highly vascularized malignant tumor.

The term "subject" refers to any animal, preferably a mammal, preferably a human. Veterinary uses are also intended to be encompassed by this invention.

This invention relates, in general, to compositions and methods for inhibition of proliferation of cancer cells or angiogenesis or a combination thereof using VEGF antisense oligonucleotides. This invention demonstrates that a variety of cancers (e.g., Kaposi's sarcoma, ovarian, pancreatic, prostate or melanoma) exhibit autocrine VEGF activity and further that administration VEGF specific antisense oligonucleotides inhibits cancer cell proliferation and tumor growth. This invention also provides screening and prognostic/diagnostic assays, as well kits comprising the VEGF antisense oligonucleotides

### ANTISENSE OLIGONUCLEOTIDES

As described herein, the present invention provides a number of oligonucleotide sequences that specifically inhibit the synthesis of VEGF protein and thus are able to block cancer cell proliferation or tumor growth. In a preferred embodiment these oligonucleotides include Veglin-3 (AS-3) which has the following sequence SEQ ID NO: 2: 5'-TGG CTT GAA GAT GTA CTC GAT-3'. In another embodiment the oligonucleotides sequences are modified in a variety of ways, such as mixed backbone oligonucleotides which comprise both deoxy and ribo nucleotides. By way of example, Veglin-3 (AS-3)(SEQ ID NO: 2) may be synthesized as a mixed backbone oligonucleotide (AS-3m) having the following sequence: 5'-UGGCTTGAAGATGTACTCGAU-3' (SEQ ID NO : 34). In the mixed backbone the bold represents 2'O-methyl ribonucleoside. Antisense oligonucleotides can also comprise truncated fragments of such sequences. Also intented to be included are the functional equivalents of these oligonucleotides,

With the published nucleic acid sequences of the target VEGF polynucleotides (e.g, Ferrara et al., (1991) *Methods Enzymol* 198:391-405; Tischer et al (1991) *J Biol Chem* 266:11947-540) and this disclosure provided, those of skill in the art will be able to identify, without undue experimentation, other antisense nucleic acid sequences that inhibit VEGF expression. For example, other sequences targeted specifically to human VEGF nucleic acid can be selected based on their ability to be cleaved by RNAse H, or to displace the binding of the disclosed antisense oligonucleotides from a nucleic acid encoding VEGF or a portion thereof These oligonucleotides are preferably at least about 14 nucleotides in length, most preferably 15 to 28 nucleotides long, with 15- to 25-mers being the most common.

These oligonucleotides can be prepared by the art recognized methods such as phosphoramidite or H-phosphonate chemistry which can be carried out manually or by an automated synthesizer as described in Uhlmann *et al.* (Chem. Rev. (1990) 90:534-583). The oligonucleotides may be composed of ribonucleotides, deoxyribonucleotides, or a combination of both.

Modified antisense nucleic acid sequences may also be utilized in the methods of the subject application. The oligonucleotides of the invention may also be modified in a number of ways without compromising their ability to hybridize to VEGF mRNA. The antisense oligonucleotide may be modified at any point in the sequence, by way of example, the ologonucleotide may be modified all along the length of the sequence, and/or in the 5' position or 3' position and/or at a select nucleotide or nucleotides. Preferred modifications include, but are not limited to, modifications which facilitate the entry of the nucleic acid sequence into a cell or modifications which protect the nucleic acid sequence from the environment (e.g., endonucleases).

Additionally, the oligonucleotides may be modified to contain other than phosphodiester internucleotide linkages between the 5' end of one nucleotide and the 3' end of another nucleotide in which the 5' nucleotide phosphodiester linkage has been replaced with any number of chemical groups. Examples of such chemical groups include alkylphosphonates, phosphorothioates, phosphorodithioates, alkylphosphonothioates, phosphoramidates, phosphate esters, carbamates, acetamidate, carboxymethyl esters, carbonates, methyl phosphonate, borane phosphonate, alpha anomer phosphodiester and phosphate triesters. Other modifications to the sugar moieties may include N3' phospormaidate, 2'0 alkyl RNA, and morpholino phosphordiamidate. In a preferred embodiment, the phosphodiester linkage has been replaced with a phosphothioate. Oligonucleotides with these linkages can be prepared according to known methods (see, *e.g,* Uhlmann *et al* (1990) Chem. Rev. 90:543-583). The term oligonucleotides also encompasses heterpolymers with totally distinct backbone structures such as polyamide nucleic acids (Nielsen, P.E. (1999), Curr. Opin. Struct. Biol, 9:353-7)

In one embodiment the oligonucleotides of the invention are modified to be composed of ribonucleotides and deoxyribonucleotides with the 5' end of one nucleotide and the 3' end of another nucleotide being covalently linked to produce mixed backbone oligonucleotides (e.g., U.S Patent Nos.: 5,652,355; 5,264,423, 5,652,356, 5,591,721). The mixed backbone oligonucleotides may be of varying length preferably being at least about 14 nucleotides in length, most preferably 15 to 28 nucleotides long, with 15- to 25-mers being the most common. The mixed backbone oligonucleotide may be any combination of ribonucleotides and deoxyribonucleotides. By way of example, the mixed backbone oligonucleotide may comprise a contigous stretch of deoxynucleotides (e,g., about 14 to about 8) flanked on either side by ribonucleotides (eg., about 2 to about 4). The phosphodiester bond may be replaced with any number of chemical group such as, for example, phosphothioate. By way of example, Veglin-3 (AS-3)(SEQ ID NO: 2) may be synthesized as a mixed backbone oligonucleotide (AS-3m) having the following sequence: 5'-UGGCTTGAAGATGTACTCGAU-3' (SEQ ID NO.: 34). Also contemplated are modified oliganucleatidesoligonucleatides which are the functional equivalent of 5'-**UGGC**TTGAAGATGTACTCGAU-3' (SEQ ID No.: 34).

The preparation of these and other modified oligonucleotides is well known in the art (reviewed in Agrawal *et al.* (1992) *Trends Biotechnol.* 10:152-158). The antisense nucleic acid sequence may be modified at any point in the sequence, for example, all along the length of the nucleic acid sequence and/or in the 5' position and/or in the 3' position. For example, nucleotides can be covalently linked using art-recognized techniques such as phosphoramidate, H-phosphonate chemistry, or methylphosphoramidate chemistry (*see, e.g.,* Uhlmann *et al* (1990) Chem. Rev. 90:543-584; Agrawal *et al.* (1987) Tetrahedron Lett. 28:(31):3539-3542); Caruthers *et al.* (1987) Meth. Enzymol, 154:287-313; U.S. Pat. No. 5,149,798), Oligomeric phosphorothioate analogs can be prepared using methods well known in the field such as methoxyphosphoramidite *(see, e.g.,* Agrawal *et al.* (1988) Proc. Natl. Acad. Sci. (USA) 85:7079-7083) or H-phosphonate *(see, e.g ,* Froehler (1986) Tetrahedron Lett. 27:5575-5578) chemistry. The synthetic methods described by Bergot *et al* (J. Chromatog. (1992) 559:35-42) can also be used. Oligonucleotides of the invention may also have modified sugars, including pendant moieties on the 2' position, and modified nucleobases, including propynyl modified bases, as well as other nonnatural bases with suitable specificity.

Preferred modifications include, but are not limited to, modifications which facilitate entry of the nucleic acid sequence into the cancer cell or modifications which protect the nucleic acid sequence from the cellular environment. Examples of such modifications include, but are not limited to, replacement of the phosphodiester bond with a phosphorothioate, phosphorodithioate, methyl phosphonate, phosphoramidate, phosphoethyl triester, butyl amidate, piperazidate, or morpholidate linkage to enhance the resistance of the nucleic acid sequence to nucleases, replacement of the phosphate bonds between the nucleotides with an amide bonds (e.g., peptide nucleic acids which are nucleobases that are attached to a pseudopeptide backbone), incorporation of non-naturally occurring bases partially or along the whole length of the nucleic acid sequence (e,g., U.S. Patent Nos. 5,192,236; 5,977,343; 5,948,901; 5,977,341) to enhance resistance to nucleases or improve intracellular absorption, or incorporation of hydrophobic substitutes such as cholesterol or aromatic rings, or polymers to the nucleic acid sequences to facilitate passage through the cellular membrane (e.g., U.S. Patent Nos. 5,192,236; 5,977,34.3; 5,948,901; 5,977,341,)

Generally, sequences which are the functional equivalent of the antisense oligonucleotides are capable of inhibiting VEGF as assessed in the assays described herein below. By way of example, IC₅₀ concentration of the antisense as assessed in a cell proliferation using, for example, the Kaposi's sarcoma cell line KSY-1 (ATCC, #CRL -11448) ranges from between about 0.5 to about 5,0 M or between about 1.0 to about 2.5 M or between about between about 1.5 to about 2.0 M, most preferably at less than or about equal to 1.5 M (see Example 9 and Table 1). Preferably such antisense oligonucleotides are derived from the coding region 261-281 (Leung et al (1989) Science 246: 1306-1309). Particularly preferred functional equivalents of the modified antisense oligonucleotides which localizes in the cell nucleus without manipulation (e.g., use of cationic lipids, permeabilizing agents).

The antisense nucleic acid sequences may impair the activity of a gene in a variety of ways and via interaction with a number of cellular products. Examples include, but are not limited to, the hydrolysis action catalyzed by RNAse H, the formation of triple helix structures, interaction with the intron-exon junctions of pre-messenger RNA, hybridization with messenger RNA in the cytoplasm resulting in an RNA-DNA complex which is degraded by the RNAas H enzyme, or by blocking the formation of the ribosome-mRNA complex and thus blocking the translation, or antisense peptides or proteins produced from the sequence of VEGF antisense, inhibit VEGF function or regulate its activity.

### ANIMAL MODEL SYSTEM

The antisense oligonucleotides may be evaluated first in animal models. The safety of the compositions and methods of treatment is determined by looking for the effect of treatment on the general health of the treated animal (weight change, fever, appetite behavior etc.) monitoring of generalized toxicity, electrolyte renal and hepatic function, hematological parameters and function measurements, Pathological changes may be detected on autopsies.

Any animal based (e.g., recombinant and non-recombinant) model systems may be used to assess the *in vivo* efficacy of the VEGF antisense oligonucleotides and to provide effective dosage ranges. For example, the relevance of the cell culture findings to the ability of the antisense oligonucleotides of the invention to be used for the treatment of a variety of cancers was confirmed by performing experiments *in vivo* in a mouse model of KS, melanoma and prostate and ovarian (see Examples 5 and 15), Tumors were implanted in immunodeficient mice were treated only for a short period and the growth of the tumor was studied for several additional days. The antisense oligonucleotides blocked the growth of the *tumor in vivo.*

### DISEASES

The VEGF antisense oligonucleotide or the equivalents thereof may be used to inhibit abnormal cellular proliferation. The VEGF antisense oligonucleotides therefor have numerous therapeutic applications in a variety of diseases including, but not limited to, diseases involving abnormal proliferation of cells, such as vascular endothelial cells (e.g., pathological angiogenesis or neovascularization). Such diseases include, but are not limited to, proliferative retinopathy (diseases of the eye in which proliferation of the blood vessels cause visual loss), macular degeneration, collagen vascular diseases, skin diseases such as psoriasis, pemphigus, diabetic retinopathy, cancers and precancerous conditions. Examples of cancer that may be treated by administration of the antisense oligonucleotides include, but are not limited to, ovarian cancer, breast cancer, pancreatic cancer, prostate cancer, melanoma, Kaposi's sarcoma, lung cancer, colon cancer, kidney cancer, prostate cancer, brain cancer, or sarcomas.

Administration of the antisense oligonucleotides serves to ameliorate, attenuate or abolish the abnormal proliferation of cells in the subject. Thus, for example, in a subject afflicted with cancer, the therapeutic administration of one or more of the antisense oligonucleotides serves to attenuate or alleviate the cancer or facilitate regression of cancer in the subject. Also contemplated is administration of the antisense oligonucleotides to a subject prior to any clinical signs of disease. Examples of such individuals includes, but is not limited to, subjects with a family history of a disease such as cancer, subjects carrying a deleterious genetic mutation or subjects at risk of disease reoccurrence.

Provided below are descriptions of non-limiting exemplary cancers that may be treated by the compositions and methods described herein.

### Kaposi's sarcoma

KS cells express all members of the VEGF family, as well as the receptors for VEGF and VEGF-C (Flt-4).Kaposi's sarcoma (KS) is the most common tumor seen in patients with HIV-1 infection (Lifson *et al.,* 1990; Reynolds, P *et al.,* 1993). KS causes significant morbidity and mortality through involvement of the skin and visceral organs. While the etiologic agent, if any, is unknown, substantial knowledge has been gained regarding the factors regulating the growth of tumor cells (Reynolds *et al.,* 1993). Kaposi's sarcoma most frequently presents as skin lesions (Lifson *et al.,* 1990). Mucosal (oral cavity) involvement is the second most common site of disease, occurring on the palate and gums and can cause tooth loss, pain and ulceration. Lymph node involvement is common with KS. However, the precise frequency is not known due to the lack of routine lymph node biopsies.

Visceral involvement occurs frequently, (in nearly 50% of the cases) especially in patients with advanced disease (Laine, L. *et al.,* 1987). Advanced gastrointestinal (GI) KS can cause enteropathy, diarrhea, bleeding, obstruction and death. Pulmonary involvement is common and significant pulmonary KS occurs in nearly 20% of the cases (Laine *et al.,* 1987; Gill, P.S. *et al.,* 1989). The symptoms vary from no symptoms to dry cough, exertional dyspnea, hemoptysis and chest pain. Pulmonary function studies may show varying degree of hypoxemia. The overall survival of patients with symptomatic pulmonary KS is less than 6 months (Gill *et al,* 1989). While the skin, lung, and GI tract are common sites of disease, nearly every organ can be involved with KS, including liver, spleen, pancreas, omentum, heart, pericardium, etc.

Phenotypic studies to define the cell of origin of KS have been performed extensively. KS spindle cells express phenotypic features of mesenchymal cells and share some markers with endothelial cell, vascular smooth muscle cells, and dermal dendrocytes. The markers shared with endothelial cells include lectin binding sites for *Ulex europeaus* Agglutinin-1 (UFA-1), CD34, EN-4, and PAL-E. The expression of several factors markers in human umbilical vein endothelial cells (HUVEC), AIDS-KS cells and trans differentiated HUVEC was confirmed by histochemistry and RT-RCR message analysis for expression of IL-6, IL-8, GM-CSF, TGF- etc.

AIDS-KS spindle cell isolation has allowed the determination of factors secreted by the tumor cells and their effects on the tumor cell itself Both IL-1 and IL-6 are produced by tumor cells. Further, the inhibition of their effects either through blocking their binding to the cognate receptors (IL-1 receptor antagonist, soluble IL-1 receptor) or inhibition of gene expression through antisense oligonucleotides (for IL-6) inhibits the growth of tumor cells More importantly, both IL-1 and IL-6 induce VEGF expression Thus endogenous production of these factors may in part be responsible for high levels of VEGF production by KS cells

The hallmark of KS is the aberrant and enhanced proliferation of vascular structures. Various angiogenic factors have been isolated for their ability to enhance endothelial cell proliferation and migration *in vitro.* Analysis of AIDS-KS cells has revealed the expression of basic fibroblast growth factor (bFGF) and vascular endothelial cell growth factor (VEGF). The latter is a secreted molecule with capability to induce capillary permeability, a prominent feature of a subset of AIDS-KS, Inhibition of VEGF expression may have therapeutic efficacy in KS. In addition, the isolation of several members of the VEGF family reveals that there is a redundancy and modulation of VEGF function. It is thus conceivable that the inhibition of VEGF alone may be active as a therapeutic strategy to inhibit tumor growth, while inhibition of several or all members of this family may be more effective.

The treatment of AIDS-related Kaposi's sarcoma is palliative. Localized KS can be managed with local therapy including radiation therapy, Radiation therapy produces local toxicity and has a cumulative dose limiting toxicity. Other options for the cosmetic treatment of localized disease include cryotherapy, photodynamic therapy, intralesional vinblastine, and intralesional sclerosing agents, all of which result in local toxicity or pigmentation which may at times be worse than the lesions itself. Progressive KS especially with local complications of pain, edema, and ulceration and symptomatic visceral KS, requires therapy which will result in rapid response. Systemic cytotoxic chemotherapy is the only treatment modality that produces rapid response The frequency of response however depends on the agent, dose, and schedule. The response to therapy varies from 25% to over 50% The most active agents include vinca alkaloids (vincristine, vinblastine), etoposide, anthracyclines and bleomycin. Combination therapies are more active than single agent treatments However, the majority of cytotoxic agents cannot be administered for a prolonged period of time due to cumulative toxicity Treatment with cytotoxic chemotherapy is palliative and the nearly all patients relapse within weeks of discontinuation of therapy.

*In vitro* studies have shown that KS cells express VEGF at high levels. In addition, VEGF receptors, VEGFR-1 and VEGFR-2 (Flt-1 and KDR), were shown to be expressed in KS cell lines. Furthermore, the addition of VEGF to the KS cells was shown to enhance KS cell growth, although it was less dramatic than seen in endothelial cells. These findings for the first time showed that KS cells express functional VEGF receptors and that VEGF acts as a growth factor for KS. This is the first demonstration of any tumor cell type to use VEGF for its own growth The role of VEGF was documented after the VEGF expression was blocked in KS cells with the use of novel antisense oligonucleotides (Veglin-1 (SEQ ID NO: 1) and Veglin-3 (SEQ ID NO: 2)). These findings indicated that under the normal conditions, the VEGF produced by the tumor cells binds with the VEGF receptors and keeps the cells proliferating In addition, it was shown that the blockage of VEGF production by the novel antisense oligonucleotides (e.g., SEQ ID NOS: 1 and 2) lead to KS cell death, indicating that VEGF not only is required for the growth of the tumor cells, but also for KS cell survival. These findings were then confirmed in the primary tumor tissues showing that VEGF and VEGF receptors are expressed in the tumor, while the normal adjoining tissue biopsies did not show expression of either VEGF or VEGF receptors.

The invention also provides methods for treating Kaposi's sarcoma with inhibition of VEGF at therapeutic doses. Specifically, this invention demonstrates that KS can be lessened and that further tumor growth and spread can be blocked with the use of specific VEGF inhibitors, antisense oligonucleotides This invention also details the parenteral administration of antisense VEGF inhibitors encapsulated in liposomes.

### Ovarian Cancer

Ovarian cancer can be separated into three major entities: epithelial carcinoma, germ cell tumors and stromal carcinomas. About 90% of the ovarian carcinomas are epithelial in origin, and the vast majority are diagnosed in postmenoposal women *(Parker et al.,* 1996). Epithelial cancer of the ovaries is usually detected only in advanced stages (III or IV) of the disease. The common pathway of tumor progression in ovarian carcinoma is via peritoneal dissemination, and the progressive accumulation of ascites is frequent with or without malignant tumor cells in the peritoneal fluid. It has been reported that ovarian carcinomas express VEGF mRNA and VEGF protein (Abu-Jaedeh *et al.,* 1996; Yamamoto S, *et al.,* 1997), VEGF is known to be produced by various solid tumors of epithelial origin and is thought to be involved in microvascular angiogenesis. In a recent study, Yamamoto and coworkers found that strong VEGF expression plays an important role in the tumor progression of ovarian carcinoma (Yamamoto S. *et al ,* 1997),

### Pancreatic Cancer

Pancreatic carcinoma is the fifth leading cause of death from cancer. At the time of detection, pancreatic carcinoma has generally spread beyond curative surgery. Furthermore, other therapies such as radiation or chemotherapy have limited value. The vast majority of patients with pancreatic cancer die within 3-6 months following diagnosis. Thus other therapeutic strategies including inhibition of VEGF are of value.

### Melanoma

Malignant melanoma belongs to the few cancers whose incidence and mortality is increasing every year. Malignant melanoma can be considered as a disorder of cell differentiation and proliferation. Normal adult melanocytes originate from a precursor melanocyte that undergoes a series of differentiation events before reaching the final end cell differentiation state (Houghton *et al.,* 1982; Houghton *et al.,* 1987).

A number of growth factors such as EGF (Singletary *et al.,* 1987), NGF (Puma *et al,* 1983), TGF (Derynk R *et al.,* 1987), PDGF (Westermark *et al,* 1986) and FGF (Moscateli *et al*., 1986) have been shown to modulate the biology of melanoma *in vitro* and also are thought to have effects on tumor transformation and progression in the animal model. The clinical importance of these growth factors is as yet undetermined. VEGF and VEGF receptor expression have been detected on two melanoma cell lines (WW94 and SW1614) but data on human tumor tissue is not available.

### Prostate carcinoma

Prostate carcinoma is the most common form of cancer in men over 50 with no curative therapy available after of failure of surgery or radiation therapy. The tumor is regulated by testosterone and its metabolites. VEGF is elevated in tumor tissue. Testosterone induces VEGF expression and thus may in part regulate prostate cancer by inducing VEGF. Inhibition of VEGF is thus of particular alone or in combination with other therapies.

### EFFECTIVE AMOUNTS

An effective amount or therapeutically effective of the antisense oligonucleotides or functional equivalents thereof to be administered to a subject in need of treatment may be determined in a variety of ways. By way of example, the antisense oligonucleotides to be administered may be chosen based on their effectiveness in inhibiting the growth of cultured cancer cells for which VEGF is an autocrine growth factor. Examples of such cell lines include, but are not limited to Kaposi's sarcoma cell lines and ovarian, pancreatic, prostate and melanoma cancer cell lines, By way of example, the oligonucleotides are able to inhibit the proliferation of the Kaposi's sarcoma cells at IC₅₀ concentrations between about 0.1 to about 100M, or between about 0.2 to about 50M, most preferably between about 0.5 to about 2.5 M or between about or between about 1 to about 5M or 1.5 to about 2.0 M, more preferably at less than about 1.5 micromolar (uM). A particularly preferred technique for determining the concentration of antisense oligonucleotide capable of inhibiting proliferation of a Kaposi's sarcoma cell line is the method outlined in Examples 3 and 9 using KS cells.

Effective concentrations of antisense oligonucleotides can be determined by a variety of techniques other than inhibition of cultured cells, such as Kaposi's sarcoma cells. Such assays can be calibrated to correspond to the data provided, for example, in Table 1. Another suitable assay that can be used is the determination of the effect of the antisense oligonucleotide on mRNA levels in a cell, such as described in Example 10. In one embodiment, antisense oligonucleotides are capable of reducing mRNA levels for one or more forms of VEGF by a factor of about 1.5 or more. In another embodiment, the antisense oligonucleotide is capable of reducing the mRNA levels of 2 or more forms of VEGF by a factor of about 2 or more.

By way of example, a general range of suitable effective dosage that may be used is about a concentration in the serum of about between about 0.5 to between about 10M. The daily dose may be administered in a single dose or in portions at various hours of the day. Initially, a higher dosage may be required and may be reduced over time when the optimal initial response is obtained. By way of example, treatment may be continuous for days, weeks, or years, or may be at intervals with intervening rest periods. The dosage may be modified in accordance with other treatments the individual may be receiving. However, the method of treatment is in no way limited to a particular concentration or range of the antisense oligonucleotides or functional equivalents thereof and may be varied for each individual being treated and for each derivative used.

One of skill in the art will appreciate that individualization of dosage may be required to achieve the maximum effect for a given individual. It is further understood by one skilled in the art that the dosage administered to a individual being treated may vary depending on the individuals age, severity or stage of the disease and response to the course of treatment. One skilled in the art will know the clinical parameters to evaluate to determine proper dosage for the individual being treated by the methods described herein. Clinical parameters that may be assessed for determining dosage include, but are not limited to, tumor size, alteration in the level of tumor markers used in clinical testing for particular malignancies. Based on such parameters the treating physician will determine the therapeutically effective amount of antisense oligo nucleotides or functional equivalents thereof to be used for a given individual. Such therapies may be administered as often as necessary and for the period of time judged necessary by the treating physician.

While it is possible for the composition comprising the antisense oligonucleotides or functional equivalents thereof be administered in a pure or substantially pure form, it is preferable to present it as a pharmaceutical composition, formulation or preparation.

### PHARMACEUTICAL COMPOSITIONS

The formulations of the present invention, are for both veterinary and human use, comprises one or more of the antisense oligonucleotides or functional equivalents thereof above, together with one or more pharmaceutically acceptable carriers and, optionally, other active agents or therapeutic ingredients. The carrier(s) must be "acceptable" in the sense of being compatible with the other ingredients of the formulation and not deleterious to the recipient thereof. The characteristics of the carrier will depend on the route of administration. Such a composition may contain, in addition to the one or more oligonucleotides and carrier, diluents, fillers, salts, buffers, stabilizers, solubilizers, and other materials well known in the art. The formulations may be prepared by any method well-known in the pharmaceutical art.

The pharmaceutical composition of the invention may also contain other active factors and/or agents which enhance inhibition of VEGF expression or which will reduce neovascularization. For example, combinations of synthetic oligonucleotides, each of which is directed to different regions of the VEGF mRNA, may be used in the pharmaceutical compositions of the invention. The pharmaceutical composition of the invention may further contain other active agents such as, nucleotide analogs such as azidothymidine, dideoxycytidine, dideosyinosine, and the like or taxol or Raloxifene and the like. Such additional factors and/or agents may be included in the pharmaceutical composition to produce a synergistic effect with the synthetic oligonucleotide of the invention, or to minimize side-effects caused by the synthetic oligonucleotide of the invention. Conversely, the synthetic oligonucleotide of the invention may be included in formulations of a particular anti-VEGF or anti-neovascularization factor and/or agent to minimize side effects of the anti-VEGF factor and/or agent. Alternatively the methods and compositions described herein may be used as adjunct therapy.

In a preferred formulation, the pharmaceutical composition of the invention may be in the form of liposomes in which the synthetic oligonucleotides of the invention is combined, in addition to other pharmaceutically acceptable carriers, with amphipathic ag ents such as lipids which exist in aggregated form as micelles, insoluble monolayers, liquid crystals, or lamellar layers which are in aqueous solution. Suitable lipids for liposomal formulation include, without limitation, monoglycerides, diglycerides, sulfatides, lysolecithin, phospholipids, saponin, bile acids, and the like. One particularly useful lipid carrier is lipofectin. Preparation of such liposomal formulations is within the level of skill in the art, as disclosed, for example, in Szoka *et al.*, Ann. Rev. Biophys. Bioeng. 9:467 (1980), U.S. Pat. Nos. 4,235,871, 4,501,728, 4,837,028; the text Liposomes, Marc J. Ostro, ed., Chapter 1, Marcel Dekker, Inc., New York (1983), and Hope *et al.,* Chem, Phys. Lip. 40:89 (1986), all of which are incorporated herein by reference. The pharmaceutical composition of the invention may further include compounds such as cyclodextrins and the like which enhance delivery of oligonucleotides into cells, as described by Zhao *et al* (Zhao Q, Temsamani J, Agrawal S (1995) Use of cyclodextrin and its derivatives as carriers for oligonucleotide delivery. *Antisense Res. Dev.* 5(3): 185-92), or slow release polymers.

The antisense oligonucleotides may be formulated as an aqueous composition s of the present invention are comprised of an effective amount of the antisense oligonucleotide, either alone or in combination with another agent (for example, but not limited to, a chemotherapeutic agent) Such compositions will generally be dissolved or dispersed in a pharmaceutically acceptable carrier or aqueous medium.

The antisense oligonucleotides the present invention can be formulated for parenteral administration, *e.g.*, for injection *via* the intravenous, intramuscular, sub-cutaneous, intratumoral or intraperitoneal routes. The preparation of an aqueous composition that contains a antisense oligonucleotide alone or in combination with another agent as active ingredients will be known to those of skill in the art in light of the present disclosure. Typically, such compositions can be prepared as injectables, such as liquid solutions or suspensions. Solid forms, that can be formulated into solutions or suspensions upon the addition of a liquid prior to injection, as well as emulsions, can also be prepared.

When oral preparations are desired, the component may be combined with typical carriers, such as lactose, sucrose, starch, talc magnesium stearate, crystalline cellulose, methyl cellulose, carboxymethyl cellulose, glycerin, sodium alginate or gum arabic among others.

In certain cases, the formulations of the invention could also be prepared in forms suitable for topical administration, such as in creams and lotions. These forms may be used for treating skin-associated diseases, such as various sarcomas.

Additional pharmaceutical methods may be employed to control the duration of action. Controlled release preparations may be achieved through the use of polymer to complex or absorb the proteins or their derivatives. The controlled delivery may be exercised by, for example, selecting appropriate macromolecules known in the art, incorporating the one or more antisense oligonucleotides either alone or in combination with other active agents into particles of polymeric material (e.g., polyesters, polyamino acids etc)or entrapping these materials in microcapsules prepared, for example, by coacervation techniques or by interfacial polymerization.

Preferred formulation is an aqueous solution given parenterally. Liposomal or lipid emulsion is another preferred method to enhance the activity. Oral formulations may allow prolonged use with greater convience.

### ROUTES OF ADMINISTRATION

Upon formulation, solutions will be administered in a manner compatible with the dosage formulation and in a therapeutically effective amount and a variety of dosage forms. An effective concentration of such antisense constructs or oligonucleotides may be administered orally, topically, intraocularly, parenterally, intranasally, intravenously, intramuscularly, subcutaneously, transdermally or by any other effective means. In addition, one or more oligonucleotide may be directly injected in effective amounts by a needle.

The formulations are easily administered in such as the type of injectable solutions described above, with even drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous, intraperitoneal, oral, intercranial, cerebrospinal fluid, pleural cavity, occular, or topical (lotion on the skin) administration. In this connection, sterile aqueous media which can be employed will be known to those of skill in the art in light of the present disclosure.

The antisense oligonucleotides formulated by the methods described herein may be delivered to the target cancer cells or any cells characterized by inappropriate cellular proliferation by a variety of methods. Examples include, but are not limited to, introducing the antisense nucleic acid of the present invention into expression vector such as a plasmid or viral expression vector Such constructs may be introduced into a cell, preferably a cancer cell, by calcium phosphate transfection, liposome (for example, LIPOFECTIN)-mediated transfection, DEAE Dextran-mediated transfection, polybrene-mediated transfection, or electroporation. A viral expression construct may be introduced into a cell, preferably a cancer cell, in an expressible form by infection or transduction. Such viral vectors include, but are not limited to, retroviruses, adenoviruses, herpes viruses and avipox viruses.

Likewise, antisense oloigonucleotides may be also be introduced into cancer cells by a variety of methods. Examples include, but are not limited to, endoscopy, gene gun, or lipofection (Mannino, R. J. et al., 1988, Biotechniques, 6:682-690)Newton, A. C. and Huestis, W. H., Biochemistry, 1988, 27:4655-4659; Tanswell, A. K. et al., 1990, Biochmica et Biophysica Acta, 1044:269-274; and Ceccoll, J. et al Journal of Investigative Dermatology, 1989, 93: 190-194),

By way of example, antisense nucleic acid sequences, such as antisense constructs or antisense oligonucleotides may be contacted with cancer cells in a body cavity such as, but not limited to, the gastrointestinal tract, the urinary tract, the pulmonary system or the bronchial system via direct injection with a needle or via a catheter or other delivery tube placed into the cancer cells. Any effective imaging device such as X-ray, sonogram, or fiberoptic visualization system may be used to locate the target cancer cells tissue and guide the needle or catheter tube.

Alternatively, the antisense nucleic acids may be administered systemically (e.g., blood circulation, lymph system) to target cancer cells which may not be directly reached or anatomically isolated.

### KIT/DRUG DELIVERY SYSTEM

All the essential materials for inhibiting VEGF expression or for inhibiting inappropriate cellular proliferation, such as tumor cell proliferation, or for inhibiting cell viability or or angiogenesis may be assembled in a kit or drug delivery system.. One or more of the antisense oligonucleotides, optionally in combination with other agents (e.g., chemotherapeutics, cytokines, antibodies directed against VEGF etc) may be formulated into a single formulation or separate formulations. The kits may further comprise, or be packaged with, an instrument for assisting with the administration or placement of the formulation to a subject. Such an instrument may be an inhalant, syringe, pipette, forceps, measured spoon, eye dropper or any such medically approved delivery vehicle. Alternatively, the container means for the formulation may itself be an inhalant, syringe, pipette, eye dropper, or other like apparatus, from which the formulation may be administered or applied to the subject or mixed with the other components of the kit.

The components of the kit may be formulated in a variety of ways. For example, the components of the kit may be provided in one or more liquid solutions, the liquid solution preferably is an aqueous solution, with a sterile, aqueous solution being particularly preferred. The components of these kits may also be provided in dried or lyophilized forms. When reagents or components are provided as a dried form, reconstitution generally is by the addition of a suitable solvent, which may also be provided in another container means. In a preferred embodiment, the oligonucleotides of the invention may be formulated as liposomes by methods known in the art.

The kits of the invention may also include an instruction sheet defining administration of the antisense oligonucleotides. The kits of the present invention also will typically include a means for containing the vials in close confinement for commercial sale such as, e.g., injection or blow-molded plastic containers into which the desired vials are retained. Other instrumentation includes devices that permit the reading or monitoring of reactions.

### 6. EXAMPLES

### Materials and Methods

Antibodies used include p-130 and Tie- 1 antibodies. Antibody p130 is an affinity-purified rabbit polyclonal antibody raised against a peptide corresponding to amino acids 1120 - 1139 mapping at the carboxy terminus of p130 of human origin. Antibody Tie-1 is an affinity-purified rabbit polyclonal antibody raised against a peptide corresponding to amino acids 1121 - 1138 mapping at the carboxy terminus of the precursor form of Tie-1 of human origin.

*Isolation of KS cells* AIDS-KS-derived spindle cell strains were isolated from primary tumor tissues as described previously (Nakamura *et al* 1988). Cells were cultured continuously in 75 cm² flasks coated with 1.5% gelatin, in KS medium consisting of the following: RPMI 1640 (Life Technologies), 100 U/mL penicillin, 100 ug/mL streptomycin, 2 mM glutamine, essential and nonessential amino acids, 10% fetal bovine serum (FBS, Life Technologies), and 1% Nutridoma-HU (Boehringer Mannheim). The primary isolates were characterized to determine their phenotype using an immunofluorescent assay. The markers expressed include endothelial cell markers; UEA-1 binding sites, EN-4, PALE; smooth muscle cell specific markers including vascular smooth muscle cell specific alpha actin; macrophage specific marker including CD14. Neoplastic cell line KSY-1 is propagated similarly and has a similar phenotype.

### Example 1

### Expression of VEGF- and VEGF-C receptors (flt-4) by KS cells

*In vitro* studies showed that KS cells express all members of the VEGF family at high levels. *Flt-1* and KDR mRNA expression was assayed in KS cell line (KSYI), HUVEC, normal skin and KS tumor tissue from an HIV+ patient, T1 (fibroblast), 23-1 {B-lymphama) and HUT-78 (T cell lymphoma). Equal amounts of RNA were reverse transcribed to generate cDNA. cDNAs were subjected to Flt-1 and KDR specific PCR amplifications (500 and 700 bp products respectively) (Fig. 3A) using paired primers, or as a control, cDNAs from all samples were subjected to -actin specific PCR amplification (548 bp product)(Fig. 3B). VEGF-C receptor (flt-4) expression was examined in a similar manner (Fig. 4).

### Example 2

### Expression of VEGF mRNA and production of VEGF protein by KS cells

VEGF mRNA expression was analyzed in several AIDS-KS cell lines. Preferably, 15 ug of total RNA from KSC10, KSC29, KSC13, KSC59 and KSY1, KSC10, HUVEC and AoSM (Fig. 1A) were electrophoresed, blotted and hybridized to the human VEGF cDNA (Fig. 1A top) and ß-actin probe (Fig. 1 A bottom). Supernatants from equal numbers of cells from KSY1, KSC10, AoSM, HUVEC and T1 were collected after 48 hours-and analyzed for VEGF protein by ELISA (Fig. 1B).

### Example 3

### Effect of VEGF antisense oligonucleotides on KS cell growth

KS cells were treated with VEGF antisense AS-1 (Veglin-1; SEQ. ID NO: 1), AS-3 (Veglin-3; SEQ. ID NO: 2), and the scrambled oligonucleotide at concentrations ranging from 1 to 10 M. The scrambled oligonucleotide used in these and subsequent experiments has the following sequence: (SEQ ID NO: 33) 5 '- TAC GTA GTA TGG TGT ACG ATC -3'. Cell proliferation was measured on day 3 (Fig, 6A), Data represent the mean ± standard error of assays performed in triplicate. Figure 6E demonstrates the effect of rhVEGF on the growth of KS and HUVEC cells. Cells were seeded at 1 X 10⁴ cells per well in 24 plates and treated with rhVEGF (1 to 10 ng/mL) for 48 hours. Cell counts were performed and the results represent the mean ± SD of an experiment performed in quadruplicate (Fig. 6E). rhVEGF abrogates the effect of VEGF antisense on AIDS-KS cell growth. KS cells were seeded at a density of 1 x 10⁴ cells per well in 24 well plates. Cells were treated with 1 and 10 M of AS-3 (Veglin-3) alone or with rhVEGF(10 ng/mL) on day 1 and day 2. Cell proliferation was measured after 72 hours. The data (Fig. 6F) represent the mean standard deviation of two experiments performed in quadruplicate. As shown by the results summarized in Figure 6, incubation of AIDS-KS cells for 3 days with antisense oligonucleotides results in a dose dependent inhibition of KS cell growth, as measured by cell count. In contrast, the sense oligonucleotides did not result in significant inhibition ofKS cell growth. These findings indicate that VEGF is an autocrine growth factor for KS cells.

### Example 4

### Specificity of VEGF antisense oligonucleotides

Antisense oligonucleotides to various coding regions of the human VEGF gene were synthesized and phosphorothioate modified to reduce degradation. Equal number of cells were seeded in 24 well plates. The molar concentration-dependent potency of VEGF antisense oligonucleotides for inhibition of growth ofKS cells (KSC-10, KSC -59) was examined in the cell proliferation assays after exposure of the cells on day and 2, and cell counts performed on day 3. Viable cell counts were determined by Coulter counter. Each value is the mean + SE of assays performed in triplicate. The controls included scrambled phosphorothioate modified oligonucleotides. In addition, the control experiments included cell lines including T-cell lines (HUT-78), B-cell lines (23-1), smooth muscle cells (AoSM), endothelial cells (HUVEC) and fibroblast (T1). Two antisense oligonucleotides tested in this experiment showed inhibition of KS cell lines, while several others had no significant effect. These oligonucleotides AS-1 and AS-3 also are referred to as SEQ ID NO: 1 and SEQ ID NO: 2. It is also notable that SEQ ID NO:1 and SEQ ID NO: 2 had no significant effect on the growth of various control cell lines, such as B cell lines, T cell lines and fibroblast cell lines.

Cells were seeded at equal density and treated with Veglin-1 (SEQ ID NO:1) or Veglin-3 (SEQ ID NO:2), or scrambled oligonucleotides (at 0, 1, 5 & 10 M), followed by a cell count (Figs. 6B, 6C and 6D) and extraction of total cellular RNA. Total RNA was isolated from AIDS-KS cells treated with various concentrations of AS-1/Veglin-1 (SEQ ID NO: 1) (Fig. 7A), AS-3/Veglin-3 (SEQ ID NO: 2) (Fig. 7B) and scrambled oligonucleotide (SEQ ID NO: 33) (Fig 7C). Total RNA was reverse transcribed to generate cDNA. PCR was carried out for VEGF and b-actin. Upper panel shows PCR products of 535 and 403 bp corresponding to VEGF,2S and VEGF,6S mRNA species of VEGF. Lower panels show the 548 bp PCR product of -actin. NT= No treatment; M= Molecular size marker, 25-41 and 18-33 represent the number of PCR cycles. The results demonstrate that AS-1/Veglin-1 and AS-3/Veglin-3 specifically reduce the accumulation of VEGF,2S and VEGF,6S mRNA species in a dose-dependent manner. Figure 7D illustrates that these VEGF oligonucleotides inhibit the production of VEGF protein in KS cells. The supernatants ofKS cells treated with AS-3 (Veglin-3) and scrambled VEGF antisense oligonucleotide were collected at 48 hr and VEGF protein was quantitated by ELISA. The results represent the mean ± standard deviation of two separate experiments done in duplicate.

### Example 5

### Inhibition of tumor growth by VEGF oligonucleotides

VEGF antisense oligonucleotide effects on tumor growth were studied in nude mice. KS-Y1 cells (1 x 10⁷) were inoculated subcutaneously in the lower back of Balb/C/Nu+/NU+ athymic mice. AS-1/Veglin-1 (SEQ ID NO:1), AS-3/Veglin-3 (SEQ ID NO:2), Scrambled (S) (SEQ ID NO: 33) VEGF oligonucleotides and diluent (PBS) were injected intra-peritoneally daily for five days (day 2 to 6). Mice were sacrificed on day 14 and tumor size was measured. Data represent the mean ± standard deviation of 10 mice in each group. Figure 8 illustrates the marked reduction in tumor growth as a result of treatment with AS- (SEQ ID NO: 1) or AS-3 (SEQ ID NO: 2). Similar experiments done on human melanoma tumor cells (M21) implanted in mice show marked reduction in tumor growth (Fig. 13). Experiments using human pancreatic carcinoma cell lines implanted in the pancreas of mice also showed tumor reduction, decrease in tumor spread, ascites and improved survival. In addition the serum and ascites VEGF levels were reduced to normal levels with AS-3.

### Example 6

### Liposomal encapsulation of VEGF antisense oligonucleotides

KS cells were treated with oligonucleotides encapsulated in neutral liposomes at various concentrations on day 1 and day 2 and the cell count was performed on day 3. Cell proliferation was measured 72 hours after start of treatment. The data represent the mean ± standard deviation of two experiments performed in quadruplicate. Liposomal encapsulation increased the apparent potency of the VEGF antisense oligonucleotides. Over 50% reduction in the cell growth was observed at concentration 50 fold below that required for free oligonucleotides *(cf* Fig. 6F, Fig. 9, bottom panel) Furthermore scrambled oligonucleotides at the same concentrations had no inhibitory effects (Fig. 9, top panel).

### Example 7

### Effect o f VEGF on KS cell survival

In addition, the effect of antisense oligonucleotides (AS-3) on KS cell survival was studied. KS cells were treated with various concentrations of oligonucleotides, The DNA was extracted and separated on agarose gel. As illustrated in Figure 10 antisense oligonucleotides at concentrations of 1 uM and above showed evidence of cell death through the mechanism of programmed cell death, also called apoptosis (Fig. 10 left panel). Scrambled oligonucleotides (SEQ ID NO:30) had no effect at concentrations of up to 10 uM (Fig. 10 right panel). This example shows that VEGF is not only an autocrine growth factor for KS cells, but is also necessary for cell survival.

### Example 8

### Effect af Flk-1/KDR and Flt-4 antibodies on KS cell growth

Figure 11A illustrates that VEGFR-2 (Flk-1/KDR) and VEGFR-3 (Flt-4) antibodies inhibit KS cell growth in a dose-dependent manner. A synergistic effect was observed when they are administered in combination. A similar effect was observed on the receptors, *i.e* antibodies to VEGFR-2 (Flk-1) and VEGFR-1 (Flt-1) induced apoptosis in a dose-dependent manner, with an additive effect when both were combined (Fig. 11B). In contrast, antibodies to another endothelial cell receptor tyrosine kinase which also is expressed on KS cells had no effect. *The in vivo* activity of VEGF receptor VEGFR-2 (Flk-1) has been shown in vivo. Relative to the controls, VEGFR-2 (Flk-1) antibody treated mice bearing KS tumor had markedly reduced tumor growth (Fig. 12).

### EXAMPLE 9

### Use of Antisense Oligonucleotides to Inhibit Cultured KS, Ovarian Carcinoma and Melanoma Cells

### Cell Proliferation Assay

The immortalized KS cell lines KS Y-1 and KS-SLK, were grown in wells coated with 1.5% gelatin in KS medium consisting of RPMI-1640 (Life Technologies, Gaithersburg MD), 100 U/ml penicillin, 100 mg/ml streptomycin, 2 mM glutamine, essential and non-essential amino acids, 10% fetal bovine serum (FBS: Life Technologies), and 1% Nutridoma-HU (Boehringer Mannheim, Indianapolis IN). The Kaposi's Sarcoma cell line KS Y-1 is available from ATCC (CRL-11448) and is the subject of US patent 5,569,602, The Kaposi's sarcoma cell line KS-SLK is available from Dr. E. Rubinstein, Chaim Sheba Medical Center, Tel-Hashomer, Israel. Human umbilical vein epithelial cells (HUVEC) (Clonetics, San Diego CA) were grown in medium containing epidermal growth factor and according to the instructions of the supplier. T1 fibroblasts; ovarian carcinoma Hoc-7 and Hey; human melanoma A375, 397 and 526 cell lines were maintained in RPMI 1640 medium supplemented with 10% FBS and antibiotics as above. The ovarian carcinoma cell lines Hoc-7 and Hey were obtained from Dr. Donald Buick, University of Toronto, Canada. The melanoma cell line A375 was obtained from the American Type Culture Collection (ATCC number CRL-1619), The melanoma cell lines 397 and 523 were obtained from Dr. Steven Rosenberg, Surgery Branch, Division of Cancer Treatment; National Cancer Institute, National Institutes of Health, Bethesda, MD, All cells were seeded at a density of 1,0 x 10⁴ cells/well in 24-well plates in appropriate growth medium on day 0. After allowing the cells to attach overnight, cells were treated with varying concentrations (1 to 10 µM) of the VEGF antisense oligonucleotide on days 1 and 3, On day 5 cell growth was assayed using 3-[4,5-dimethylthiazol-1-yl]-2,5-diphenyltetrazolium bromide (MTT). Wells were treated with 0,5 mg/ml MTT in 90% isopropanol, 0.5% SDS and 40 mM HCl. Developed color was read at 490 nm in an ELISA plate reader (Molecular Devices, CA) using isopropanol as a blank.

Antisense oligonucleotides corresponding to regions of VEGF mRNA were synthesized by standard chemical techniques. The oligonucleotides were synthesized as phosphorothioate without further modification. IC₅₀ values were determined using the cell proliferation assay as described above and are reported in Table 1.

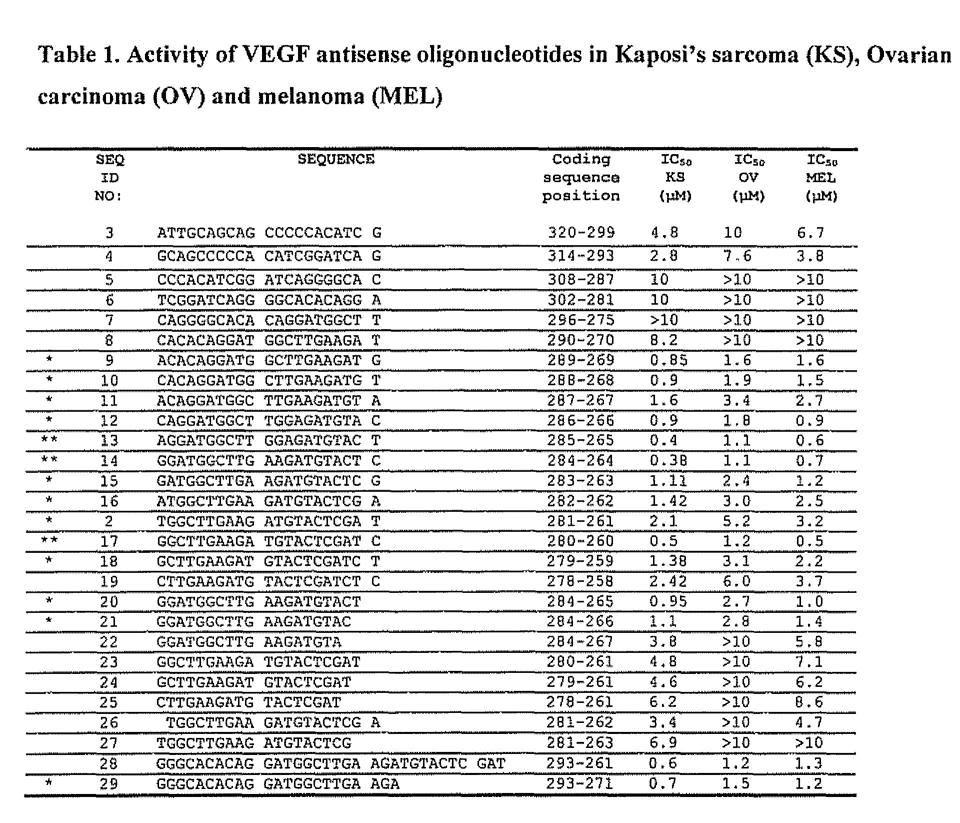

Nucleotide numbering shown in the fourth column is from the translation start site of VEGF-165 isoform as published in: Leung DW, Cachianes G, Kuang W-J, Goeddel DV, and Ferrara N. (1989) "Vascular endothelial growth factor is a secreted angiogenic mitogen." *Science* **246**:1306-1309. The antisense molecules are represented, as per the convention, in the 5' 3' orientation. Antisense molecules are complements to the coding strand of the DNA, which also by convention is represented and numbered 5' 3'. Nucleic acids anneal to strands with opposing polarity, therefore the numbers in the fourth column, which represent the gene sequence appear 3'5' (higher to lower). IC₅₀ values indicate the concentration of antisense oligonucleotide necessary to inhibit cell proliferation by 50%.

### EXAMPLE 10

### Effect of Antisense Oligonucleotides on Expression of VEGF-A, -C and -D

KS Y-1 cells were seeded at a density of 1 x 10⁴ per well in gelatin-coated plates on day 0. The cells then were treated individually with antisense oligonucleotides SEQ ID NOS: 3-29, at various concentrations (0, 1, 5, and 10 uM) on day 1. Cells were harvested and total RNA was extracted on day 3, cDNAs were synthesized by reverse transcriptase using a random hexamer primer in a total volume of 20 ul (Superscript, Life Technologies Inc.). Five microliters of the cDNA reaction were used for PCR using gene-specific primers for i) VEGF-A, ii) VEGF-C and iii) VEGF-D. Each PCR cycle consisted of denaturation at 94 °C for 1 min, primer annealing at 60 °C for 2 min, and extension at 72 °C for 3 min. The samples were amplified for 41 cycles, and 5 ul aliquots were removed from the PCR mixtures after every 4 cycles starting at cycle 25. Amplified product was visualized on a 1.5 % agarose gel containing ethidium bromide. All samples analyzed for VEGF-A, -C or -D expression also were analyzed for beta-actin expression to confirm the integrity and quantity of the RNA. Table 2 shows the effect of antisense oligonucleotides SEQ ID NO:2 and SEQ ID NO:14 on the expression of various VEGF members corrected for beta-actin amplification.

### Table 2. Quantitation of mRNA levels in response to antisense oligonucleotides.

Table 2 demonstrates the effects of various antisense oligonucleotides on the expression of VEGF protein family members. AS-3/Veglin-3 (SEQ ID NO: 2) produced a dose-dependent decline in VEGA-A mRNA levels. AS-3/Veglin-3 had no significant effect on VEGF-C, VEGF-D or PIGF expression. In contrast, SEQ ID No: 14 produced dose-dependent declines in the mRNA levels of VEGF-A, -C, and -D. This antisense molecule lowered VEGF-A mRNA levels from 2.7-3 fold at I uM and 4.6-6.3 fold at 5 uM. Furthermore the levels of VEGF- C and VEGF-D declined to similar magnitude and were 3-fold reduced at 1 uM and 6-fold reduced at 5 uM concentrations. There was no significant effect on PIGF. Neither of these oligonucleotides produced a decline in mRNA levels of beta-actin, a house keeping gene.

**Table 2.**

| **Quantitation of mRNA levels in response to antisense oligonucleotides. Fold Decline in mRNA levels** | | | | | |
|---|---|---|---|---|---|
| | VEGF-A | VEGF-C | VEGF-D | PIGF | ß-actin |
| AS-3/Veglin-3/SEQ ID NO:2 | | | | | |
| 1 uM | 1.6 | none | none | none | none |
| 5 uM | 3.2 | none | none | none | none |

| SEQ ID NO: 14 | | | | | |
|---|---|---|---|---|---|
| 1 uM | 2.7-3.0 | 3 | 3 | none | none |
| 5 uM | 4.6-3.2 | 6 | 6 | none | none |

The ability of an antisense oligonucleotide to inhibit cell growth may be dependent on its ability to inhibit multiple forms of VEGF. Table 3 shows the relative effects of antisense oligonucleotides directed towards VEGF on VEGF,-A, -C, and -D gene expression. Particular, high affinity sequences are capable of inhibiting multiple forms of VEGF. Those antagonists showing the largest inhibition are marked with two asterisks. Other antagonists showing broad activity against multiple forms of VEGF are marked with a single asterisk. Using these data, one of skill in the art can select an appropriate oligonucleotide sequence for inhibiting a specific form of VEGF, or for inhibiting growth of tumor cells, a sequence that broadly inhibits multiple VEGF forms.

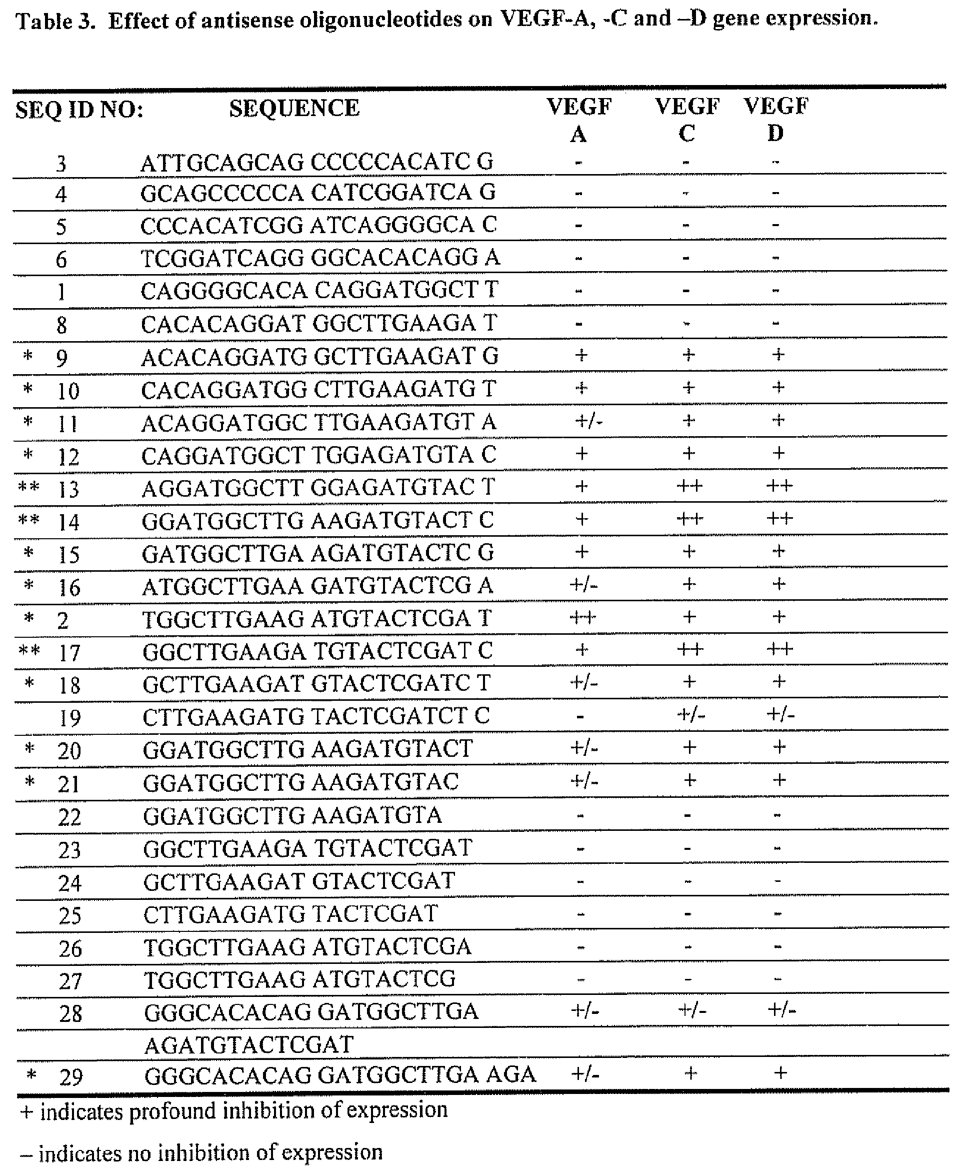

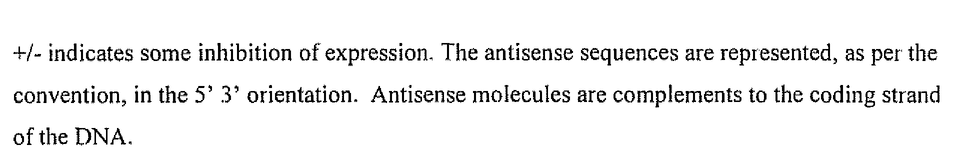

### EXAMPLE 11

### Effect ofantisense Oligonucleotides on Pancreatic Cancer Cells

Vascular endothelial growth factor (VEGF) is overexpressed in human pancreatic cancer (PaCa). Previous studies suggest that VEGF acts not directly on PaCa cells, but as paracrine stimulator of tumor neoangiogenesis. This study investigated VEGF production/ expression in human pancreatic cancer cells and evaluated the effect of a VEGF antisense oligonucleotide on in-vivo growth and angiogenesis of human PaCa in an orthotopic nude mouse model.

*In-vitro* Two human PaCa cell lines (AsPC-1 poorly differentiated; HPAF-2, moderately to well differentiated) were evaluated/tested for VEGF mRNA transcripts by RT-PCR. VEGF secretion in cell culture supernatant was assessed by ELISA. Both PaCa cell lines expressed VEGF mRNA and secreted VEGF protein (AsPC-1: 420539 pg/10⁶ cells; HPAF-2: 812364 pg/10⁶ cells).*In*-*vivo*: VEGF antisense oligonucleotide (AS-3/Veglin-3, SEQ ID NO:2) were synthesized with phosp horothioate modification, 1 mm³ fragments of sc. PaCa donor tumors were orthotopically implanted into the pancreas of nude mice. Animals received either AS-3 (10 mg/kg, daily) or the vehicle ip. for 14 weeks. Volume of primary tumor (TU-Vol.), metastic spread (Met-Score), and VEGF-expression in serum (VEGF_{S}) and ascites (VEGF_{A}) were determined at autopsy. Microvessel density (MVD) was analyzed by immunohistochemistry in CD31-stained tumor sections. The results of these *in vivo* studies are shown in Table 4.

**Table 4.**

| Results of AS-3/Veglin-3 treatment. | | | | |
|---|---|---|---|---|
| *=p<0.05 vs. Control | AsPC-1 | | HPAF-2 | |
| | Control | AS-3 | Control | AS-3 |
| TU-Vol. (mm³) | 1404 149 | 1046 81 | 3829 594 | 860 139* |
| Met-Score (pts.) | 16.7 0.9 | 6.5 0.8* | 8.3 1.5 | 2.5 0.2* |
| Survival (n / n) | 1/8 | 6/8* | 4/8 | 7/8 |
| VEGF_{S} (pg/ml) | 59.5 5.8 | 26.6 1.1* | 192.3 41.2 | 38.3 6.1* |
| VEGF _{A} (pg/ml) | 1190 88 | No ascites | 1405 97 | no ascites |
| MVD (/0.74 mm²) | 64.1 4.4 | 33,2 23* | 76.4 6.0 | 24,1 2.5* |

Human PaCa cells secrete a high level of biologically active VEGF *in vitro.* VEGF-antisense therapy reduces VEGF secretion and tumor neoangiogenesis *in vivo,* thereby reducing tumor growth and metastasis, and improving survival. Metastasis seems to be particularly susceptible to VEGF-AS therapy. None of the AS-3 treated animals developed ascites, suggesting that vascular permeability was also reduced by inhibiting VEGF expression in PaCa cells.

### EXAMPLE 12

### Expression of VEGF and VEGF Receptors in Human Tumor Cell Lines

**Cell lines and Reagents:** The cell lines T1, HuT 78, A375, LNCaP, U937 and HL-60 were all obtained from the ATCC (Manassas, VA). Other cell lines were obtained from colleagues at the University of Southern California; M21 (Bumol, T. F. & Reisfeld, R. A. (1982) Proc Natl Acad Sci U S A 79:1245-9) was from P. Brooks, 526 from J, Weber, Hey and Hoc-7 from L. Dubeau and Panc-3 was from D. Parekh. KS Y-1 has been described previously (Lunardi et al., (1995) J. Natl cancer Institute 87:974-81). VEGFR-1 polyclonal antibody (C-17), VEGFR-2 polyclonal antibody (C-1158) were from Santa Cruz Biotechnology (Santa Cruz, CA). Recombinant human VEGF was purchased from R & D Systems (Minneapolis, MN).

**Preparation of CDNA and RT**-**PCR:** Total RNA was prepared from 1 x 10⁵ cells. Complementary DNAs were synthesized by reverse transcription (RT) using a random hexamer primer in a total volume of 20 1 (Superscript II, Life Technologies, Gaithersberg, MD). Five microliters of the cDNA reaction were amplified by PCR as previously described (Masood, R. et al., (1997) Proc Natl Acad Sci U S A 94, 979-84). Each PCR cycle consisted of denaturation at 94°C for 1 min, primer annealing at 60°C for 2 min and extension at 72°C for 3 min. The samples were amplified for 30 cycles. Amplified product was visualized on 1.5 % agarose gels containing ethidium bromide. The integrity and quantity of cDNA was confirmed for all samples by amplification of -actin. Primers used to detect cDNA are listed below in Table 5A.

**Flow Cytometry:** Flow cytometry was used to analyze the expression of cell surface molecules. All cell lines (KS Y-1, M21, Hey, T1, U937) were seeded at a density of I x 10⁶ per T75 flask in appropriate culture media. Adherent cells (KS Y-1, M21, Hey, T1) were harvested on the following day using a rubber policeman. Cells grown in suspension (U937, HL-60, A6876, P.3HR1) were transferred to 12 x 15 mm round-bottomed centrifuge tubes. Viable cell counts were determined by trypan blue dye exclusion. Cells were incubated with antibodies (Flt-1, Fik-1, control serum all from Santa Cruz Biotechnology, Inc.) followed by anti-rabbit FITC conjugate (Sigma). The cells were washed twice with ice cold phosphate buffered saline (PBS) after each incubation. Cell pellets were suspended in 1 ml of PBS and analyzed with a FACScan flow cytometer (Becton Dickinson). The data are presented as mean fluorescence intensity ratios (MFIRs) (mean fluorescence intensity with Ab of interest/mean fluorescence intensity with control isotype specific rabbit IgG). Negative controls were cells incubated with anti-rabbit FITC, with no prior exposure to receptor-specific antibodies.

VEGF production was assessed in a variety of human tumor cell lines. Human melanoma (M21), human ovarian carcinoma (Hey and Hoc-7), and human prostate carcinoma (LNCaP) all secrete high levels of VEGF into the culture medium (Table 6). This is in contrast to a human T-cell leukemia cell line (HuT-78) and human fibroblasts (T1), which do not have detectable VEGF. We also determined VEGF mRNA levels by RT-PCR in these cell lines and others, including Panc3 representative of pancreatic carcinoma, Hey-7 and Hoc representative of ovarian carcinoma, A375 and 526, representative of melanoma. All cell lines tested, except the T1 fibroblasts, expressed VEGF (Table 6).

The expression of VEGF receptors (VEGFR-1/Flt-1 and VEGFR-2/Flk-1) was also examined. A number of human tumor cell lines derived from melanoma, ovarian carcinoma and pancreatic carcinoma showed VEGF receptor expression by several different methods including RT-PCR, immunocytochemistry, and flow cytometry. The results are summarized in Figure 15 and Table 6. Flow cytometry and RT-PCR also showed that an erythroid leukemia cell line, HL-60, and T-cell leukemia, HuT 78, did not express VEGFR-1 or -2 (Fig. 15A). U937, a monocytoid cell line expressed high levels of VEGFR-1 (Table 6) but not VEGFR-2. The co-expression of VEGF and its receptors in some of these tumor cell lines raised the possibility of autocrine growth factor activity. This activity could be tested by blocking expression of the ligand, VEGF.

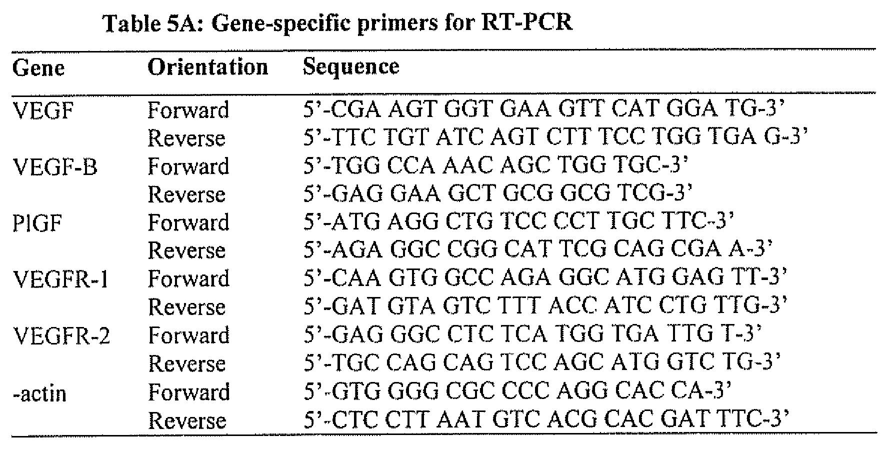

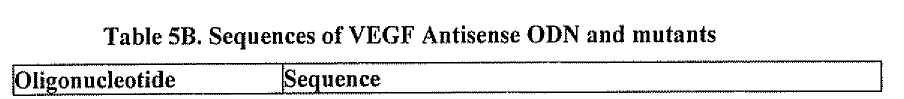

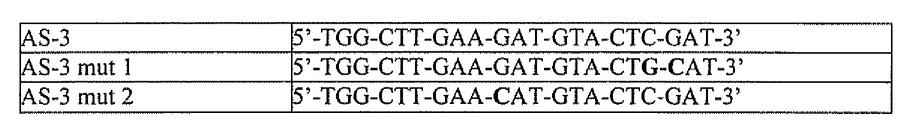

**Table 6:**

| **Expression of VEGF and its receptors in tumor cell lines** | | | | |
|---|---|---|---|---|
| **Cell line** | **Type** | **VEGF (pg/10**^{**6**} **cells)*** | **VEGFR-2 (Flk-1)** | **VEGFR-1 (Flt-1)** |
| KS Y-1 | Kaposi's sarcoma | +(625) | + | + |
| M21 | Melanoma | +(487) | + | + |
| A375 | Melanoma | + | + | + |
| 526 | Melanoma | + | + | + |
| Hey | Ovarian carcinoma | +(4)9) | + | + |
| Hoc-7 | Ovarian carcinoma | + (550) | + | + |
| PANC3 | Pancreatic carcinoma | + | + | + |
| LNCaP | Prostate carcinoma | + (719) | + | - |
| U937 | Pro-monocytoid | +(1476) | - | + |
| HL-60 | Erythroid leukemia | - | - | - |
| HuT 78 | T cell leukemia | - | - | - |
| T1 | Fibroblast | - | - | - |

| | | | | |
|---|---|---|---|---|
| *Cells were cultured for 48 h. VEGF levels in the supernatants were measured by ELISA (R&D Systems) | | | | |

### EXAMPLE 13

### VEGF-AS3 Specifically Blocks VEGF Expression

**Test Oligonucleotides:** VEGF-specific ODN, referred to here as AS-3 and complementary to VEGF mRNA (261 to 281) (Leung, D. W, et al., (1989) Science 246,1306-9), and two mutants of AS-3 were synthesized with or without 5' fluorescein tag (Operon technologies, Alameda, CA) as shown in table 5. Mutated bases are shown in bold face. Mixed back bone derivative of AS-3 (named AS-3m) **5'-UGGC**TTGAAGATGTACT**CGAU**-3' and a control 21-mer mixed backbone ODN, referred to here as 'scrambled', 5'-UCGCACCCATCTCTCTCCUUC -3', were synthesized, purified and analyzed as previously described Agrawal, S. et al., (1997) Proc Natl Acad Sci U S A 94, 2620-5. Four nucleotides at the 5'- end and four nucleotides at the 3'- end are 2'- O-methylribonucleosides (represented by bold face letters); the remaining are deoxynucleosides. For both mixed-backbone oligonucleotides, all internucleotide linkages are phosphothioate. The purity of the oligonucleotides was shown to be greater than 90% by capillary gel electrophoresis and PAGE, with the remainder being n-1 and n-2 products. The integrity of the internucleotide linkage was confirmed by ³¹PNMR.

**Immunofluorescence Studies:** It was next demonstrate that the AS-ODNs described here enters the cells, 5'- Fluorescein-tagged AS-ODNs listed in Table 5 were synthesized (Operon Technologies, Alameda CA). KS Y-1 cells were seeded onto chamber slides (Nunc) at a density of 10,000 cells per well in serum containing medium and allowed to attach overnight. The medium was replaced with serum free medium and the cells were exposed to fluorescein-tagged AS-3m, AS-3m mut 1 or AS-3m mut 2 for four hours. Notably we did not use cationic lipids or permeabilizing agents to enhance uptake of the oligonucleotides, At the conclusion of the 4-hour incubation, the cells were washed 5 times with phosphate buffered saline (PBS). The chambers were removed and the live cells were placed under coverslips and analyzed by confocal microscopy.

**Determination of VEGF and IL-8 protein levels:** Cells were cultured in 2% FCS for these experiments. Cells were treated with various concentrations of the oligonucleotides at hr 0 and 16. The supernatants were collected at hr 24, centrifuged to remove all cell debris and stored at -70°C until analysis using ELISA kits (R&D Systems, Minneapolis, MN) according to the manufacturer's instructions. Levels of VEGF detected were corrected for cell numbers. Tumor tissues from the in vivo experiments on tumor growth were lysed and the levels of VEGF protein were determined using both the human VEGF ELISA kit and a mouse VEGF ELISA kit (also from R & D Systems). Levels of VEGF detected were corrected for total protein.

AS-3 and mutants with either mutation of one or two nucleotides (Table 5B) (all were phosphothioate modified) were thus tested for their effect on the viability of cell lines that show VEGF dependent autocrine growth factor activity. KS Y1 cells cultured in 1% FCS, were treated with ODNs on days 1 and 3, and the cell viability was assessed by MTT assay on day 5. A dose dependent loss of viability was observed with AS-3 while both mutants had marked reduction in this activity (Figure 16A). AS-3 mut 2, which has a single base change resulted in a 60% loss in efficacy at a concentration of 2.5 uM AS-ODN. Results were similar for AS-3 mut1.

To confirm the specificity of the ODN activity, equal number of KS Y1 cells were allowed to adhere in medium containing 1% FCS Cells were treated with various concentrations of the oligonucleotides at hr 0 and 16. The supernatants were collected at hr 24 and analyzed for either VEGF or IL-8. VEGF was nearly completely inhibited at 10 uM of AS-3, while the effects of either of the two mutants were substantially less. Thus in short term experiments, a higher dose of the ODN was required for complete inhibition of VEGF and the activity was sequence dependent.

To determine that the inhibition of VEGF was not related to non-specific effect, same supernatants were studied for the production of other secreted proteins. KS Y1 cells produce significant amounts of IL-8, which was not affected by the parent compound AS-3, or either of the two mutants. Thus the activity of AS-3 is highly specific for inhibition of VEGF and is sequence dependent.

In order to determine that the reduced activity of the mutants was not related to the failure of cellular uptake, fluorescein labeled ODNs were studied by immunofluorescence. 5'-Fluorescein-tagged AS-ODNs were synthesized (Operon Technologies, Alameda CA). KS Y-1 cells were seeded onto chamber slides (Nunc) at a density of 10,000 cells per well in serum containing medium and allowed to attach overnight. The medium was replaced with serum free medium and the cells were exposed to fluorescein-tagged AS-3m, AS-3m mut1 or AS-3m mut 2 at various concentrations for four hours. Notably we did not use cationic lipids or permeabilizing agents to promote cellular uptake of the oligonucleotides. At the conclusion of the 4-hour incubation, the chambers were removed and the live cells were placed under coverslips and analyzed by confocal microscopy. Figure 15C shows overlay images of the fluorescein fluorescence and phase contrast. Fluorescent signal is detectable in the cells of all samples treated with the lowest concentration of the ODN tested (1uM), and appears to be localized to the nucleus. The cellular uptake and nuclear localization was not affected by mutation of one or two nucleotides. These data when taken together show that VEGF-AS-3 is highly specific inhibitor of VEGF and that the activity is sequence dependent. Also tested was fluorescien VEGF-AS3 and the mutants in melanoma (M21) and ovarian carcinoma cell line (Hey). All three ODN's were taken up by these cells.

Also tested was a mixed backbone oligonucleotide (MBO) corresponding to the previously described AS-3 sequence (Fig. 17A). The sequence of AS-3m is complementary to VEGF mRNA and contains a number of mismatches for the other VEGF family genes (Fig. 17B) so we tested the specificity of its activity in KS Y-1 cells. Treatment of KS Y-1 cells, which express all VEGF family members, with AS-3m led to a dose-dependent inhibition of VEGF mRNA compared to untreated levels at 5 M (Fig. 18A). In contrast, in the presence of 5 M AS-3m the levels of VEGF-B and PIGF (VEGF related proteins) and the unrelated -actin message did not change significantly, indicating that the effect is specific. Having shown that AS-3m significantly inhibited VEGF message, it was next shownthat it inhibited VEGF protein production *in vitro.* Incubation of both M21 melanoma and Hey ovarian carcinoma cell lines with AS-3m resulted in a dose-dependent drop in the levels of VEGF protein in the culture supernatants (Fig. 18B). No significant effects were seen using the scrambled MBO. Thus the mixed back bone derivative of AS-3 retains the activity to inhibit VEGF expression and protein production.

### EXAMPLE 14

### VEGF-AS directly inhibits tumor cell proliferation in vitro

**Cell proliferation assay:** Cells were seeded at a density of 1 x 10⁴ per well in 48-well gelatin coated plates on day 0 in appropriate growth media containing 2% fetal calf serum (FCS), except for KS Y-1 where 1% FCS was used. On the following day, the media was changed and cells were treated with various concentrations (1-10 M) of oligonucleotides. Medium was changed and treatment was repeated on day 3. On day 5, viability was assessed using 3-(4,5-dimethylthiazol-2-yl)-2,5-diphenyltetrazolium bromide (MTT) at a final concentration of 0.5 mg/ml. Cells were incubated for 2 hr, medium was aspirated, and the cells were dissolved in acidic isopropanol (90% isopropanol, 0.5% SDS and 40 mM HCI). Optical density was read in an ELISA reader at 490 nm using the isopropanol as blank (Molecular Devices, CA).

KS cell lines are derived from endothelial cell lineage, and that the process of transformation is associated with activation of VEGF. Like endothelial cells, KS cells express VEGF receptors. Inhibition of VEGF expression was then shown to inhibit KS cell proliferation and viability. It has been suggested that VEGF receptors function only in the context of endothelial cells, since induced expression of VEGF receptors using expression vectors failed to establish VEGF mediated signaling in certain non-endothelial lineage cell types. However, the data presented here demonstrates that several of the cell tumor cells from diverse tumor types express both VEGF and VEGF receptors. Thus, it appears that in the case of neoplastic transformation, cells may acquire the ability to not only express VEGF but also to acquire VEGF receptors and signaling pathways specific to VEGF.

Next it was examined if the inhibition of VEGF using VEGF-AS3 or its derivative could influence cell viability in the context of VEGF loop. The data shows a range of response to VEGF inhibition, Notably the cell lines that show most inhibition of cell viability were those that expressed both VEGF and VEGF receptors. Melanoma and ovarian carcinoma cell lines showed the most response and were similar to KS cell line (KSY1). In sharp contrast the cell lines that failed to show response were erthroleukemia (HL-60), HUT-78 and fibroblast (T1) cell lines all of which lack VEGF and VEGF receptor expression. Results were similar for VEGF-AS3 or VEGF-AS3m (Fig. 19A, left panel). Scrambled MBO derived ODN had no significant effect except for minimal toxicity at higher dose levels in selected cell lines (Fig. 19A, right panel). The role of VEGF in cell viability was further confirmed by the addition of recombinant VEGF, which nearly completely abrogated the effect of AS-3m in M21 (Fig. 19B, left panel) and Hey cells (Fig, 5B, right panel). These results are of clinical significance since we and others have shown that a substantial portion of primary tumor cells express VEGF and VEGFR-1/R-2 (Herold-Mende, C. et al., (1999) Lab Invest 79, 1573-82),

### EXAMPLE 15

### Inhibition of Tumor Growth In Vivo

**In Vivo studies:** Human tumor cell lines KS Y-1, M21, and Hey (2 x 10⁶ cells) were injected subcutaneously in the lower back of 5-week old male Balb/C Nu⁺/nu⁺ athymic mice. In the first protocol treatment consisted of daily oral administration of AS-3m or scrambled MBO or diluent (PBS) begun the day following tumor cell implantation and continued for two weeks. Dosing was 10 mg/kg in 1001 PBS by gavage. In the second protocol, designed to test tumor regression, the cells were implanted and the xenograft was allowed to establish for 5 days before treatment was initiated. Treatment consisted of daily intraperitoneal injection of AS-3m (1, 5 or 10 mg/kg in a total volume of 100 1) or diluent. Taxol (1.25 or 2.5 mg/kg) treatment, where indicated was by intraperitoneal injection on days 5 and 12. Tumor growth in mice was measured three times in a week. Mice were sacrificed at the conclusion of the study, Tumors were collected and analyzed for VEGF levels. All mice were maintained in accord with the University of Southern California institutional guidelines governing the care of laboratory mice.

It was shown that VEGF PS-ODN AS-3 specifically inhibits growth of KS Y-1 tumor xenografts in mice (Masood et al (1997) PNAS). The same model was used to determine if the mixed backbone oligonucleotide AS-3m may be orally available. Daily oral administration of AS-3m over the course of two weeks resulted in the near complete inhibition of KS Y-1 tumor xenograft growth (Fig. 20A, left panel). The growth of KS was completely blocked in some mice while the tumor size was minimal in others. Mice that did not have appreciable tumor were then observed without therapy. The recurrence of the tumor was observed in all mice within four weeks (data not shown). Similar treatment regimen of human melanoma M21 tumor xenografts by daily oral administration of AS-3m resulted in tumor volumes of less than 20% of the controls (Fig. 20A, right panel) when the treatment was initiated the day following tumor implant. A dose dependent activity was also established if the treatment was delayed for five days allowing tumor to establish (Figure 19B left panel), a dose range of 1, 5 and 10 mg/kg showed tumor growth inhbition of 20%, 68% and over 80% respectively. In addition, an additive effect was observed when VEGF-AS3m was combined with low dose taxol (Figure 20B right panel), illustrating that the combined treatment regimes were more potent than either agent used alone. It is apparent that the effects of Taxol and AS-3m at the doses used here are additive. In vivo studies of VEGF-AS3 using ovarian carcinoma cell line (Hey) also showed marked response.

### EXAMPLE 16

### Effect of AS-3m on VEGF levels in vivo

Human tumor xenografts (human ovarian cell line hey) were harvested 24 hours after the last dose of therapy and tumor lysates were prepared. VEGF levels were quantitated and adjusted for total protein. A dose-dependent inhibition of both human (tumor derived) and mouse (host derived) VEGF was observed on treatment with AS-3m. In a representative experiment approximately 60% reduction in the levels of both human and mouse VEGF was observed after daily dose of 10mg/kg (Table 7). The nucleotide sequence of VEGF-AS3 has a stretch of 17 nucleotides that are homologous to the mouse VEGF coding region (Fig. 17C) and thus may explain the targeting of mouse VEGF as well.

**Table 7:**

| **Levels of human and mouse VEGF in antisense treated tumor-(hey) bearing mice** | | |
|---|---|---|
| | VEGF (pg/mg protein; mean ± S.E.M.) | |
| Treatment group | Mouse | Human |
| Control (diluent only) | 76.14 ± 17.81 | 198.29 ± 29.88 |
| 1 mg/kg AS-3m | 47,11 ± 3.47 | 175.15 ± 33.54 |
| 5 mg/kg AS-3m | 34.68 ± 4.27 | 94.71 ± 19.57* |
| 10 mg/kg AS-3m | 31.1 ± 4.05* | 81.20 ± 15.50* |

| | | |
|---|---|---|
| * *P* ≤ 0.05 | | |

### EXAMPLE 17

### VEGF-AS3 is active in Orthotopic prostate cancer model

**Orthotopic implantation of Tumor Cells:** Cultured PC-3P cells (60-80% confluent) were harvested for injection. Mice were anaesthetized with methocyflurane, and a lower midline incision was made. Tumor cells (1X10⁵/10 l) in HBSS were implanted in the dorsal prostate lobes using a dissecting microscope. The cells were injected through a 30 gauge needle using a syringe with calibrated push button controlled dispensing system. Formation of a small bullas at the injection site was required to include mice in the study. The prostate gland was returned to its natural location, and the abdominal incision was closed. Mice were treated with either the saline or the study drug beginning on day 10, Six mice were included in each group. The treated group received VEGF AS-3m at a dose of 10 mg/kg IP. daily for a period of two weeks. Mice were sacrificed on day 24 after the tumor implantation. Prostate and tumors were excised under dissecting microscope. The tissues were fixed in 10% buffered formalin, placed in OCT (Miles Laboratories, IN). Tissue sections were stained with either H&E or processed for immunocytochemistry.

**Immunohistochemistry:** Formalin-fixed tissues sections were deparaffinized and incubated with 10% goat serum at -70°C for 10 minutes and incubated with the primary rabbit antibodies against either VEGFR-1/flt-1, or VEGFR-2/Flk-1/KDR (1:100) at 40°C overnight. Isotype specific rabbit IgG was used as control The immunoreactivity for these receptors was revealed using an avidin-biotin kit from Vector Laboratories (Burlingame, CA). Peroxidase activity was revealed by the diaminobenzidine (Sigma) cytochemical reaction. The slides were then counterstained with 0.12% methylene blue or H&E.

Expression of VEGF increases with advancing prostate carcinoma and increases even further when the tumor becomes hormone independent. Prostate carcinoma can only be treated with palliative therapy if not respectable. Prostate gland stroma like other organs plays critical role tissue remodeling and tumor regulation. To determine if inhibition of VEGF had anti-tumor effect human prostate tumor cell line (PC3) was examined by direct tumor implantation of the mouse prostate gland with,. Treatment was delayed to ten days post implantation, and the treatment consisted of AS-3m daily at a dose of 1 mg/kg. Three weeks after the tumor implant the mice were sacrificed and the prostate gland was harvested for analysis. All control mice (n=6) developed tumor at the site of injection in the prostate. There was evidence of VEGF expression within the tumor cells and the stroma, and the presence of CD31 positive microvessels in the tumors by immunohistochemistry. Lymphocyte infiltration was seen predominantly around the tumor with very little if any lymphocyte migration into the tumor tissue (Fig 21A upper panel). Only two of the six treated mice showed tumor, which were relatively small (Fig 21A lower panel). The most striking finding was the presence of immune cells within the tumor. In situ characterization of infiltrating cells revealed the presence of monocytes, dendritic cells and NK cells (fig 21B upper panel). The expression of NK cytolytic proteins such as perforin and granzyme B were also localized to the region of NK cells (fig 21B lower panel). In addition, interferon inducible protein- 10 (IP-10) was also localized predominantly to the region of cellular infiltrate (fig 21 B lower panel). IP-10 is produced in response to interferon gamma and appears to regulate NK cell function and independently inhibit angiogenesis.

VEGF plays a pivotal role in vasculogenesis and angiogenesis (Plate, K. H. (1998) *Adv Exp Med Biol* 451, 57-61). This is particularly significant due to over expression of the endothelial cell mitogen VEGF in tumor cells and elevated VEGF receptors in the tumor vasculature. Furthermore elevated VEGF levels are associated with tumor metastasis and survival (Chan, A. S. et al., (1998) *Am J Surg Pathol* 22, 816-26; Benjamin, L. E. & Keshet, E. (1997) *Proc Natl Acad Sci* USA 94, 8761-6, Benjamin, L. E. et al., (1999) *J Clin Invest* 103, 159-65). Various inhibitors under development include monoclonal antibody to VEGF, inhibitor of VEGF receptor activation following ligand binding etc (Fong, T. A. et al., (1999) *Cancer Res* 59, 99-106; Yukita, A. et al., (2000) *Anticancer Res* 20, 155-60; Dias, S. et al., (2000) in *Proc American Assoc Cancer Res,* Vol. 41, pp, 792).

The preceding examples demonstrate that VEGF-AS3 enters the cells and localizes in the nucleus without any manipulation such as the use of cationic lipids or the use of membrane permeabilizing agents. The cellular uptake of the ODNs is highly variable and limited due to the negative charge. Furthermore it was shown that the activity is sequence dependent since VEGF-AS3 inhibited VEGF production but not other proteins such as IL-8, while mutation in one or two nucleotides had significantly reduced the ability to inhibit VEGF production without loss of cellular uptake. The specific activity was further confirmed in the cell lines that display VEGF mediated autocrine growth factor activity.

Also shown herein was that a number of tumor cell lines that produce VEGF also express VEGF receptors. These results indicate a loss of regulatory function since prolonged VEGF exposure leads to down regulation of the VEGF receptors in normal endothelial cells (Wang, D. et al., (2000) *J Biol Chem* 275, 1 5905-15911). It was also shown that the receptors are functional. Presence of VEGF autocrine growth factor activity was demonstrated in four different human tumor types including melanoma, ovarian carcinoma, pancreatic carcinoma and Kaposi's sarcoma. These cells all express VEGF, the mitogenic receptor VEGFR-2 and show impaired viability in response to VEGF ablation. The inhibition of cell viability was restored by the exogenous VEGF. Expression of VEGF receptors on tumor cells has been described previously (Herold-Mende, C. et al., (1999) *Lab Invest* 79, 1573-82), and mitogenic response to exogenous VEGF has been documented in pancreatic carcinoma, choriocarcinoma and melanoma (Itakura, J. et al., (2000) *Int J Cancer* 85, 27-34; Charnock-Jones, D. S. et al., (1994) *Biol Reprod* 51, 524-30; Liu, B. et al., (1995) *Biochem Biophys Res Commun* 217, 721-7). Without being bound by theory, the presence of autocrine growth pathways in some tumors implies that VEGF antisense therapy is acting on two levels: antiangiogenic effects on the tumor vasculature and antineoplastic effects on the tumor cell population. VEGFR-2 expression in the tumor cells may thus predict for better response to VEGF ablation.

Phosphorothioate oligodeoxynucleotides (PS-ODNs) have been used most extensively in o rder to stabilize ODNs. PS-ODNs have shown a profile of side effects such as fever, liver dysfunction, hepatomegaly, thrombocytopenia, activation of complement etc. The side effects are related to the polyanionic charge of ODNs. ODNs have also been shown to induce certain cytokines such as IL-6, IL-12, TNF-alpha etc. The induction of cytokines appear to be sequence dependent especially the presence of CpG islands. CpG islands are defined by the presence of CpG flanked by a pair of purines on the 5' end and and a pair of pyrimidine nucleotides on the 3'end induce cytokines(J. Immunology (2000) 164: 1617-1624), ODNs with CpG islands also activate B cells and monocytes. Runs of dG (G strings) can also induce non-specific effects. Nuclease resistant backbone may stimulate B cell function. VEGF-AS3 and VEGF-AS3mut1 do not contain CpG islands, or G strings, and did not show induction of inflammatory cytokines.

Derivatives of VEGF-AS3 mixed back bone ODNs in which portions of the ODNs are substituted with modified nucleoside were evaluated. VEGF-AS3 specifically contains segments (four nucleosides at each end) of 2-O-methylribonucleosides at both the 3'- and 5'-ends of PS-ODNs. A stretch of more than six to eight PS-ODNs is required to retain the Rnase I activation. The VEGF-AS3m derivative was shown to retain specificity to inhibit VEGF expression in vitro and in vivo. Antitumor activity is observed following parenteral as well as oral administration. VEGF-AS3m was also combined with chemotherapy with additive activity. In conclusion, it was shown that VEGF-AS3 is a highly specific inhibitor of VEGF, it is taken up by the cells, and is active in vivo alone, and additive or synergistic when combined with other therapies.

### 7. REFERENCES

Abu-Jaedeh GM, Faix JD, Niloff J, Tognazzi K, Manseau E, Dvarak HF, Brown LF. Strong expression of vascular permeability factor (vascular endothelial growth factor) and its receptors in ovarian borderline and malignant neoplasms Lab. Invest. 1996: 74; 1105-1115.
Agrawal *et al.* (1987) Tetrahedron. Lett. **28**:(31):3539-3542.
Agrawal *et al.* (1988) Proc. Natl. Acad. Sci. (USA) **85**:7079-7083.
Agrawal *et al.* (1992) Trends Biotechnol. **10**:152-158.
Barillari G, Buonaguro L, Fiorelli V. *et al.* Effect of cytokines from activated immune cells on vascular cell growth and HIV-1 gene expression. J Immunol 1992, 149:3727-3734,
Bayer, E.A. *et al.* (1979) *Meth Enzym.* **62**:308,
Bergot *et al* (1992) *J Chromatog* **559**:35-42.
Campbell, A.M. (1984) *Monoclonal Antibodies Technology: Laboratory Techniques in Biochemistry and Molecular Biology,* Elsevier Science Publishers, Amsterdam, The Netherlands.
Caruthers *et al.* (1987) *Meth. Enzymol.* **154**:287-313.
Chak LY, Gill PS, Levine AM, Meyer PR, Anselmo JA, Petrovich Z. Radiation therapy for Acquired Immunodeficiency Syndrome related Kaposi's sarcoma. *J. Clin. Oncol.* 1988, **62**:735-739.
Cole *et al.* (1985) in *Monoclonal Antibodies and Cancer Therapy,* Alan R. Liss, Inc., pp. 77-96.Engval, E. *et al. Immunol.* 1972, **109**: 129.
Derynck R, Goeddel DV, Ullrich A, Gutterman U, Williams RD, Bringman TS and Berger WH. Synthesis of messenger RNAs for TGF and and epidermal growth factor receptor by human tumors. Cancer Res. 1987, **47**:707-712.
Ensoli B, S. N, Salahuddin SZ, et al. AIDS-Kaposi's sarcoma-derived cells express cytokines with autocrine and paracrine growth effects. Science 1989, 94:223-226.
Froehler *Tetrahedron Lett.* 1986, **27**:5575-5578.
Garvey, J. S. *et al.* (1977) *Methods in Immunology,* 3rd ed., W. A. Benjamin, Inc., Reading, MA.
Gelman EP, Longo DL, Lane HL, *et al.* Combination chemotherapy of disseminated Kaposi's sarcoma in patients with the acquired immunodeficiency syndrome. *Am. J Med* 1987, **82**:456-459.
Gill PS, Akil B, Rarick M, Colletti P, *et al.* Pulmonary Kaposi's sarcoma: Clinical findings and results of therapy. *Am J. Med.* 1989, 87:57-61.
Gill PS, Rarick MU, Bernstein-Singer, Harb M, Espina B, Shaw V, Levine AM. Treatment of advanced Kaposi's sarcoma using a combination of Bleomycin and Vincristine. *Am J Clin. Oncol.* 1990, **13**:315-319.
Gill PS, Rarick MU, McCutchan JA, *et al.* Systemic treatment of AIDS-related Kaposi's sarcoma. Results of a randomized trial. *Am. J Med.* 1991, **19**:427-433.
Gill PS, Espina BM, Muggia F, Cabriales S, Tulpule A, Esplin JA, Liebman HA, Forssen E, Ross ME, Levine AM (1995) Phase I/II clinical and pharmacokinetic evaluation of liposomal daunorubicin. *J Clin. Oncol.* **13**:996-1003
Coding, J.W. (1976) *J Immunol Meth.* **13**: 215.
Houghton AN, Eisinger M, Albino AP, Cairncross JG, and Old LJ. Surface antigens of melanocytes and melanoma. Markers of melanocytes differentiation and melanoma subset. J *Exp. Med.* 1982, **156**:1755-1766.
Houghton AN, Real FX, Davis LJ, Cardon-Cardo C and Old LJ. Phenotypic heterogeneity of melanoma. Relation to the differentiation program of melanoma cells. *J Exp*. *Med.* 1987,**164**:812-829.
Kohler, G. and Milstein, C. (1975) *Nature* **256**: 495-497.
Kozbor, D. *et al* (1983) *Immunology Today* **4**:72.
Krown SE, Real FX, Cunningham-Rundles S, *et al.* Preliminary observations on the effect of recombinant leukocyte A interferon in homosexual men with Kaposi's sarcoma. *N Engl. J Med.* 1983, **308**:1071-1076.
Laine L, Politoske EJ, Gill PS. Protein-losing enteropathy in acquired immunodeficiency syndrome due to the intestinal Kaposi's sarcoma. *Arch. Intern. Med* 1987, **147**:1174-1175.
Lane HC, Feinberg J, Davey V, *et al.* Anti-retroviral effects of interferon-a in AIDS associated Kaposi's sarcoma. *Lancet* 1988, **2**:1218-1222.
Lassoned SC, Claurel JP, Katlama C, *et al* Treatment of acquired immunodeficiency syndrome related Kaposi's sarcoma with bleomycin as a single agent. *Cancer* 1990, **66**:1869-1872.
Laubenstein LJ, Krigel RL, Odajnk CM *et al.* Treatment of epidemic Kaposi's sarcoma with etoposide or a combination of doxorubicin, bleomycin, and vinblastine. *J. Clin. Oncol* 1984, **2**:1115-1120.
Leung DW, Cachianes G, Kuang W-J, Goeddel DV, and Ferrara N. Vascular endothelial growth factor is a secreted angiogenic mitogen. 1989, *Science* **246**:1306-1309
Lifson AR, Darrow WW, Hessol NA, O'Malley PM, Bamhart JL. Jaffe HW, and Rutherford GW. Kaposi's sarcoma in a cohort of homosexual and bisexual men. *American Journal of Epidemiology* 1990, **131**:221-231.
Louie S, Cai J, Law R *et al., Effects* of interleukin- and interleukin-1 receptor antagonist in AIDS-Kaposi's sarcoma. *J. AIDS Hum. Retrovirol.* 8:455-60.
Lutz *et al. Exp. Cell Research* 1988, **175**: 109-124.
Masood R, Husain SR, Rahman A and Gill PS. Potentiation of cytotoxicity of Kaposi's sarcoma related to immunodeficiency syndrome (AIDS) by liposome encapsulated Doxorubicin. *AIDS Res. Hum. Retroviruses* 1993, **9**:741-745.
   Masood R, Cai J, Zheng T, Smith DL, Naidu Y, Gill PS. Vascular endothelial growth factor/vascular permeability factor is an autocrine growth factor for AIDS-Kaposi sarcoma. *Proc. Natl. Acad Sci. USA* 1997, **94**:979-84
Miles SA, Rezai AR, Salazar-Gonzales JF, *et al.* AIDS-Kaposi's sarcoma derived cells produce and respond to interleukin-6. Proc Natl Acad Sci USA 1990, 87:4068.
Mintzer D, Real FX, Jovino L *et al.* Treatment of Kaposi's sarcoma and thrombocytopenia with vincristine in patients with the acquired immunodeficiency syndrome. Ann Intern Med 1985, **102**:200-202.
Moscatelli D, Preston M, Silverstein J et al. Both normal and tumor cells produce basis fibroblast growth factor. *J. Cell Physiol.* 1986, **123**:273-276.
Nair BC, Devico AL, Nakamura S, *et al.* Identification of a major growth factor for AIDS-Kaposi's sarcoma cell as Oncostatin-M. *Science* 1992, **255**:1430-1432.
Nickoloff BJ, Griffith CEM. The spindle-shaped cells in cutaneous Kaposi's sarcoma. Histologic simulators include factor Xlllz dermal dendrocytes. *Am J. Pathol.* 1989, **135**:793-800.
Parker SL, Tong T, Bolden S, Wingo PA. 1996: Cancer statistics, 1996 *Ca*: *a Cancer Journal for Clinicians.* 1996, **46**:5-27.
Puma P, Buxser SE, Watson L, Kellcher DJ and Johnson GL. Purification of the receptor for nerve growth factor from A875 melanoma cells by affinity chromatography. *J Biol. Chem.* 1983, **256**:3370-3375.
Reynolds P, Saunders LD, Layefsky ME, and Lemp GF. The spectrum of acquired immunodeficiency syndrome (AIDS)-associated malignancies in San Francisco, 1980-87. *American Journal of Epidemiology* 1993, **137**:19-30.
Russell Jones R, Spaull J, Spry C, Wilson Jones E. Histogenesis of Kaposi's sarcoma in patients with and without acquired immunodeficiency syndrome. *J Clin. Pathol* 1986, **39**:742-749,
Shweitzer VG, Visscher D. Photodynamic therapy for treatment of AIDS-related oral Kaposi's sarcoma otolaryngol. *Head Neck Surg.* 1990, **102** : 639-649.
Singletary SE, Baker FL, Spitzer G, Tucker SL, Tamosoric B, Brock WA, Ajiani JA, and Kelly AM. Biological effect of epidermal growth factor on the in vitro growth of human tumors. Cancer Res. 1987: 47;403-406.
Sternberger, L. A. *et al.* (1970) *J. Histochem. Cytochem.* **18:** 315,
Vogel J, Hinrichs SH, Reynolds RK, *et al* The HIV tat gene induces dermal lesions resembling Kaposi's sarcoma in transgenic mice. *Nature* **335:**606-611, 1988.
Volbering PA, Abrams D1, Conant M *et al.* Vinblastine therapy for Kaposi's sarcoma in acquired immunodeficiency syndrome, *Ann. Int. Med.* 1985, **103**:335-338.
Weich HA, Salahuddin SZ, Gill PS, Nakamura S, Gallo R, Folkmann J. AIDS associated Kaposi's-derived cells in long-term culture express and synthesize smooth muscle alpha-actin. *Am. J. Pathol.* 1992, **139**:1251-1258.
Weindel K, Mamme D, Welch HA: AIDS-associated Kaposi's sarcoma cells in culture express vascular endothelial growth factor. Biochem. Biophys. Res. Commun. 1992, 183,1167-1174.
Westermark B, Johnsson A, Paulsson Y, Betsholtz C, Heldin C, Herlyn M, Rodeck U, and Kaprowski H. Human melanoma cell lines of primary and metastatic origin express the genes encoding the chains of PDGF and produce a PDGF like growth factor. *Proc Natl Acad Sci USA* 1986, **83**:7197-7200.
Uhlmann *et al. Chem. Rev.* 1990, **90**:534-583.
Yamamoto S, Konishi I, Mandai M, Kuroda H, Komatsu T, Nanbu K, Sakahara H, Mori T. Expression of vascular endothelial growth factor (VEGF) in epithelial ovarian neoplasms: correlaation owith clinicopathology and patient survival, and analysis of serum VEGF levels. *British J*. *Cancer* 1997, **76**:1221-1227.
Zhao Q, Temsamani J, Agrawal S (1995) Use of cyclodextrin and its derivatives as carriers for oligonucleotide delivery. *Antisense Res. Dev.* **5**:185-92.

Although the present invention has been described in some detail by way of illustration and examples for purposes of clarity of understanding it will be obvious that certain changes and modifications may be practiced within the scope of the appended claims,

## Claims

1. A composition comprising one or more antisense oligonucleotide directed against vascular endothelial growth factor (VEGF), and wherein said one or more antisense oligonucleotide comprises the sequence of SEQ ID NO: 2, and wherein said one or more antisense oligonucleotide is modified so as to facilitate entry into a cell or confer protection from endonucleases.

2. The composition of claim 1, wherein the antisense oligonucleotide contains one or more ribonucleotides,

3. The composition of claim 1, wherein the antisense oligonucleotide contains both deoxyribonucleotides and ribonucleotides.

4. The composition of claim 3, wherein the antisense oligonucleotide is UGGCTTGAAGATGTACTCGAU (SEQ ID NO: 34).

5. The composition according to anyone of claims 1-4, wherein the antisense oligonucleotide contains one or more phosphorothioate moieties.

6. The composition according to anyone of claims 1-5, wherein the antisense oligonucleotide inhibits proliferation of cells exhibiting autocrine VEGF activity at an IC₅₀ concentration of between about 0.5 to about 2.5 micromolar.

7. The composition of claim 5, where in the IC₅₀ concentration is less than or equal to about 1.5 micromolar.

8. The composition according to anyone of claims 1-7, wherein said one or more antisense oligonucleotide is encapsulated in a liposome.

9. The composition according to anyone of claims 1-8, further comprising a chemotherapeutic, such as taxol.

10. The composition according to anyone of claims 1-9, for medical use.

11. An antisense oligonucleotide of SEQ ID NO: 2.

12. An antisense oligonucleotide of SEQ ID NO: 34.

13. The antisense oligonucleotide of claim 11 or 12, wherein the antisense oligonucleotide contains one or more phosphorothioate moieties.

14. Use of the antisense oligonucleotide according to anyone of claims 11-13, for preparing a pharmaceutical composition for inhibiting cancer proliferation, in particular proliferation of ovarian cells, Kaposki's sarcoma cells, prostate cells or pancreatic cancer cells,

15. Use of the antisense oligonucleotide according to anyone of claims 11-13, for preparing a pharmaceutical composition for inhibiting angiogenesis.

16. , Use of the antisense oligonucleotide according to anyone of claims 11-13, for preparing a pharmaceutical composition for inhibiting VEGF expression.


### Annex to the application documents - subsequently filed sequences listing

SEQUENCE LISTING

<110> Gill, Parkash
      Masood, Rizwan

<120> METHODS AND COMPOSITIONS FOR ANTISENSE
  VEGF OLIGONUCLEOTIDES

<130> VASG-PE1-003

<140> EP 04105588.0
<141> 2004-11-08

<150> EP 01942565.1
<151> 2001-01-19

<150> US 09/487,023
<151> 2000-01-19

<160> 64

<170> FastSEQ for Windows Version 4.0

<210> 1
<211> 21
<212> DNA
<213> Artificial Sequence

<220>
<223> VEGF antisense oligonucleotide

<400> 1
agacagcaga aagttcatgg t                                                21

<210> 2
<211> 21
<212> DNA
<213> Artificial Sequence

<220>
<223> VEGF antisense oligonucleotide

<400> 2
tggcttgaag atgtactcga t                                                21

<210> 3
<211> 21
<212> DNA
<213> Artificial Sequence

<220>
<223> VEGF antisense oligonucleotide

<400> 3
attgcagcag cccccacatc g                                                21

<210> 4
<211> 21
<212> DNA

<213> Artificial Sequence

<220>
<223> VEGF antisense oligonucleotide

<400> 4
gcagccccca catcggatca g                                                21

<210> 5
<211> 21
<212> DNA
<213> Artificial Sequence

<220>
<223> VEGF antisense oligonucleotide

<400> 5
cccacatcgg atcaggggca c                                                21

<210> 6
<211> 21
<212> DNA
<213> Artificial Sequence

<220>
<223> VEGF antisense oligonucleotide

<400> 6
tcggatcagg ggcacacagg a                                                21

<210> 7
<211> 21
<212> DNA
<213> Artificial Sequence

<220>
<223> VEGF antisense oligonucleotide

<400> 7
caggggcaca caggatggct t                                                21

<210> 8
<211> 21
<212> DNA
<213> Artificial Sequence

<220>
<223> VEGF antisense oligonucleotide

<400> 8
cacacaggat ggcttgaaga t                                                21

<210> 9
<211> 21
<212> DNA
<213> Artificial Sequence

<220>
<223> VEGF antisense oligonucleotide

<400> 9
acacaggatg gcttgaagat g                                                21

<210> 10
<211> 21
<212> DNA
<213> Artificial Sequence

<220>
<223> VEGF antisense oligonucleotide

<400> 10
cacaggatgg cttgaagatg t                                                21

<210> 11
<211> 21
<212> DNA
<213> Artificial Sequence

<220>
<223> VEGF antisense oligonucleotide

<400> 11
acaggatggc ttgaagatgt a                                                21

<210> 12
<211> 21
<212> DNA
<213> Artificial Sequence

<220>
<223> VEGF antisense oligonucleotide

<400> 12
caggatggct tggagatgta c                                                21

<210> 13
<211> 21
<212> DNA
<213> Artificial Sequence

<220>
<223> VEGF antisense oligonucleotide

<400> 13
aggatggctt ggagatgtac t                                                21

<210> 14
<211> 21
<212> DNA
<213> Artificial Sequence

<220>
<223> VEGF antisense oligonucleotide

<400> 14
ggatggcttg aagatgtact c                                                21

<210> 15
<211> 21
<212> DNA
<213> Artificial Sequence

<220>
<223> VEGF antisense oligonucleotide

<400> 15
gatggcttga agatgtactc g                                                21

<210> 16
<211> 21
<212> DNA
<213> Artificial Sequence

<220>
<223> VEGF antisense oligonucleotide

<400> 16
atggcttgaa gatgtactcg a                                                21

<210> 17
<211> 21
<212> DNA
<213> Artificial Sequence

<220>
<223> VEGF antisense oligonucleotide

<400> 17
ggcttgaaga tgtactcgat c                                                21

<210> 18
<211> 21
<212> DNA
<213> Artificial Sequence

<220>
<223> VEGF antisense oligonucleotide

<400> 18
gcttgaagat gtactcgatc t                                                21

<210> 19
<211> 21
<212> DNA
<213> Artificial Sequence

<220>
<223> VEGF antisense oligonucleotide

<400> 19
cttgaagatg tactcgatct c                                                21

<210> 20
<211> 20
<212> DNA
<213> Artificial Sequence

<220>
<223> VEGF antisense oligonucleotide

<400> 20
ggatggcttg aagatgtact                                                  20

<210> 21
<211> 19
<212> DNA
<213> Artificial Sequence

<220>
<223> VEGF antisense oligonucleotide

<400> 21
ggatggcttg aagatgtac                                                    19

<210> 22
<211> 18
<212> DNA
<213> Artificial Sequence

<220>
<223> VEGF antisense oligonucleotide

<400> 22
ggatggcttg aagatgta                                                     18

<210> 23
<211> 20
<212> DNA
<213> Artificial Sequence

<220>
<223> VEGF antisense oligonucleotide

<400> 23
ggcttgaaga tgtactcgat                                                   20

<210> 24
<211> 19
<212> DNA
<213> Artificial Sequence

<220>
<223> VEGF antisense oligonucleotide

<400> 24
gcttgaagat gtactcgat                                                    19

<210> 25
<211> 18
<212> DNA
<213> Artificial Sequence

<220>
<223> VEGF antisense oligonucleotide

<400> 25
cttgaagatg tactcgat                                                     18

<210> 26
<211> 20
<212> DNA
<213> Artificial Sequence

<220>
<223> VEGF antisense oligonucleotide

<400> 26
tggcttgaag atgtactcga                                                   20

<210> 27

<211> 19
<212> DNA
<213> Artificial Sequence

<220>
<223> VEGF antisense oligonucleotide

<400> 27
tggcttgaag atgtactcg                                                    19

<210> 28
<211> 33
<212> DNA
<213> Artificial Sequence

<220>
<223> VEGF antisense oligonucleotide

<400> 28
gggcacacag gatggcttga agatgtactc gat                                    33

<210> 29
<211> 23
<212> DNA
<213> Artificial Sequence

<220>
<223> VEGF antisense oligonucleotide

<400> 29
gggcacacag gatggcttga aga                                               23

<210> 30
<211> 62
<212> DNA
<213> Homo sapiens

<220>
<221> CDS
<222> (3)...(62)
<223> VEGF-A

<400> 30
ag atc gag tac atc ttc aag cca tcc tgt gtg ccc ctg atg cga tgc     47
   Ile Glu Tyr Ile Phe Lys Pro Ser Cys Val Pro Leu Met Arg Cys
    1               5                  10                  15

ggg ggc tgc tgc aat                                                62
Gly Gly Cys Cys Asn
                20


<210> 31
<211> 62
<212> DNA
<213> Homo sapiens

<220>
<221> CDS
<222> (3)...(62)
<223> VEGF-C

<400> 31
cg aca aac acc ttc ttt aaa cct cca tgt gtg tcc gtc tac aga tgt     47
   Thr Asn Thr Phe Phe Lys Pro Pro Cys Val Ser Val Tyr Arg Cys
    1               5                   10                  15

ggg ggt tgc tgc aat                                                62
Gly Gly Cys Cys Asn
                20

<210> 32
<211> 62
<212> DNA
<213> Homo sapiens

<220>
<221> CDS
<222> (3)...(62)
<223> VEGF-D

<400> 32
gt acc aac aca ttc ttc aag ccc cct tgt gtg aac gtg ttc cga tgt     47
   Thr Asn Thr Phe Phe Lys Pro Pro Cys Val Asn Val Phe Arg Cys
    1               5                   10                  15

ggt ggc tgt tgc aat                                                62
Gly Gly Cys Cys Asn
                20

<210> 33
<211> 21
<212> DNA
<213> Artificial Sequence

<220>
<223> Scrambled VEGF antisense oligonucleotide

<400> 33
tacgtagtat ggtgtacgat c                                                21

<210> 34
<211> 21
<212> DNA
<213> Artificial Sequence

<220>
<223> Mixed backbone oligonucleotides, nucleotides 1-4
      and 18-21 are 2'O methylribonucleosides

<400> 34
uggcttgaag atgtactcga u                                                21

<210> 35
<211> 21
<212> DNA
<213> Artificial Sequence

<220>
<223> Scrambled mixed backbone oligonucleotides,
      nucleotides 1-4 and 18-21 and 2'O
      methylribonucleosides

<400> 35
ucgcacccat ctctctccuu c                                                21

<210> 36
<211> 23
<212> DNA
<213> Artificial Sequence

<220>
<223> VEGF gene specific primers for RT-PCR

<400> 36
cgaagtggtg aagttcatgg atg                                              23

<210> 37
<211> 25
<212> DNA
<213> Artificial Sequence

<220>
<223> VEGF gene specific primers for RT-PCR

<400> 37
ttctgtatca gtctttcctg gtgag                                            25

<210> 38
<211> 18
<212> DNA
<213> Artificial Sequence

<220>
<223> VEGF-B gene specific primers for RT-PCR

<400> 38
tggccaaaca gctggtgc                                                    18

<210> 39
<211> 18
<212> DNA
<213> Artificial Sequence

<220>
<223> VEGF-B gene specific primers for RT-PCR

<400> 39
gaggaagctg cggcgtcg                                                    18

<210> 40
<211> 21
<212> DNA
<213> Artificial Sequence

<220>
<223> PIGF gene specific primers for RT-PCR

<400> 40
   atgaggctgt ccccttgctt c                                               21

<210> 41
<211> 22
<212> DNA
<213> Artificial Sequence

<220>
<223> PIGF gene specific primers for RT-PCR

<400> 41
agaggccggc attcgcagcg aa                                                22

<210> 42
<211> 23
<212> DNA
<213> Artificial Sequence

<220>
<223> VEGFR-1 gene specific primers for RT-PCR

<400> 42
caagtggcca gaggcatgga gtt                                               23

<210> 43
<211> 23
<212> DNA
<213> Artificial Sequence

<220>
<223> VEGFR-1 gene specific primers for RT-PCR

<400> 43
caagtggcca gaggcatgga gtt                                               23

<210> 44
<211> 24
<212> DNA
<213> Artificial Sequence

<220>
<223> VEGFR-2 gene specific primers for RT-PCT

<400> 44
gatgtagtct ttaccatcct gttg                                              24

<210> 45
<211> 23
<212> DNA
<213> Artificial Sequence

<220>
<223> VEGFR-2 gene specific primers for RT-PCT

<400> 45
tgccagcagt ccagcatggt ctg                                               23

<210> 46
<211> 20
<212> DNA
<213> Artificial Sequence

<220>
<223> Beta-actin gene specific primers for RT-PCR

<400> 46
gtggggcgcc ccaggcacca                                                   20

<210> 47

<211> 24
<212> DNA
<213> Artificial Sequence

<220>
<223> Beta-actin gene specific primers for RT-PCR

<400> 47
ctccttaatg tcacgcacga tttc                                              24

<210> 48
<211> 21
<212> DNA
<213> Artificial Sequence

<220>
<223> A mutation of the antisense oligonucleotide SEQ ID
      NO: 2

<400> 48
tggcttgaag atgtactgca t                                                 21

<210> 49
<211> 21
<212> DNA
<213> Artificial Sequence

<220>
<223> A mutation of the antisense oligonucleotide SEQ ID
      NO: 2

<400> 49
tggcttgaac atgtactcga t                                                 21

<210> 50
<211> 21
<212> DNA
<213> Homo sapiens

<220>
<221> CDS
<222> (1)...(21)
<223> VEGF-A

<400> 50
atc gag tac atc ttc aag cca                                         21
Ile Glu Tyr Ile Phe Lys Pro
 1               5


<210> 51
<211> 21
<212> DNA
<213> Homo sapiens

<220>
<221> CDS
<222> (1)...(21)
<223> VEGF-B

<400> 51
gtg gcc aaa cag ctg gtg ccc                                         21

Val Ala Lys Gln Leu Val Pro
 1               5

<210> 52
<211> 21
<212> DNA
<213> Homo sapiens

<220>
<221> CDS
<222> (1)...(21)
<223> VEGF-C

<400> 52
aca aac acc ttc ttt aaa cct                                          21
Thr Asn Thr Phe Phe Lys Pro
 1               5

<210> 53
<211> 21
<212> DNA
<213> Homo sapiens

<220>
<221> CDS
<222> (1)...(21)
<223> PIGF

<400> 53
gtg gag cac atg ttc agc cca                                          21
Val Glu His Met Phe Ser Pro
 1               5

<210> 54
<211> 21
<212> DNA
<213> Homo sapiens

<220>
<221> CDS
<222> (1)...(21)
<223> VEGF-D

<400> 54
acc aac aca ttc ttc aag ccc                                          21
Thr Asn Thr Phe Phe Lys Pro
 1               5

<210> 55
<211> 21
<212> DNA
<213> Mus musculus

<220>
<221> CDS
<222> (1)...(21)
<223> Nucleotides 288-308 of the sequence reported in
      Claffey et al (1992) J. Biol. Chem 267: 16317-2257

<400> 55
ata gag tac atc ttc aag ccg                                          21
Ile Glu Tyr Ile Phe Lys Pro
 1               5

<210> 56
<211> 20
<212> PRT
<213> Homo sapiens

<400> 56
Ile Glu Tyr Ile Phe Lys Pro Ser Cys Val Pro Leu Met Arg Cys Gly
 1               5                  10                  15
Gly Cys Cys Asn
            20

<210> 57
<211> 20
<212> PRT
<213> Homo sapiens

<400> 57
Thr Asn Thr Phe Phe Lys Pro Pro Cys Val Ser Val Tyr Arg Cys Gly
 1               5                  10                  15
Gly Cys Cys Asn
            20

<210> 58
<211> 20
<212> PRT
<213> Homo sapiens

<400> 58
Thr Asn Thr Phe Phe Lys Pro Pro Cys Val Asn Val Phe Arg Cys Gly
 1               5                  10                  15
Gly Cys Cys Asn
            20

<210> 59
<211> 7
<212> PRT
<213> Homo sapiens

<400> 59
Ile Glu Tyr Ile Phe Lys Pro
 1               5

<210> 60
<211> 7
<212> PRT
<213> Homo sapiens

<400> 60
Val Ala Lys Gln Leu Val Pro
 1               5

<210> 61
<211> 7
<212> PRT
<213> Homo sapiens

<400> 61
Thr Asn Thr Phe Phe Lys Pro
1               5


<210> 62
<211> 7
<212> PRT
<213> Homo sapiens

<400> 62
Val Glu His Met Phe Ser Pro
1               5


<210> 63
<211> 7
<212> PRT
<213> Homo sapiens

<400> 63
Thr Asn Thr Phe Phe Lys Pro
1               5


<210> 64
<211> 7
<212> PRT
<213> Mus musculus

<400> 64
Ile Glu Tyr Ile Phe Lys Pro
1               5